# EUROPEAN PATENT SPECIFICATION

(11) **EP 4 297 645 B1**
(45) Date of publication and mention of the grant of the patent: **03.07.2024**
(21) Application number: 22707091.9
(22) Date of filing: 18.02.2022
(51) Int. Cl.: A61B 5/05, A61B 5/08, A61B 5/11, A61B 5/00, G08B 21/04, H04B 7/22, H04B 17/309, H04W 4/33, G05B 15/02, G05B 19/418, H04W 84/12

(54) **FALL-CLASSIFICATION DEVICE AND ARRANGEMENT**
FALLKLASSIFIZIERUNGSVORRICHTUNG UND ANORDNUNG
DISPOSITIF ET AGENCEMENT DE CLASSIFICATION DE CHUTE

(30) Priority: 24.02.2021 US 202163152960 P; 22.03.2021 EP 21164010
(43) Date of publication of application: 03.01.2024
(73) Proprietor: Signify Holding B.V., 5656 AE Eindhoven (NL)
(72) Inventor: KRAJNC, Hugo José, 5656 AE Eindhoven (NL); DEIXLER, Peter, 5656 AE Eindhoven (NL); ROZENDAAL, Leendert Teunis, 5656 AE Eindhoven (NL); POORT, Simone Helena Maria, 5656 AE Eindhoven (NL); VAN DER HEIJDEN, Gerardus Wilhelmus Theodorus, 5656 AE Eindhoven (NL); KATARIA, Sonia, 5656 AE Eindhoven (NL)
(74) Representative: Verweij, Petronella Daniëlle
(86) International application number: PCT/EP2022/054085
(87) International publication number: WO 2022/179948

(56) References cited:
- CN-A- 103 606 248
- CN-A- 107 749 143
- CN-A- 108 614 989
- US-A1- 2019 175 074
- HU YUQIAN ET AL: "A WiFi-Based Passive Fall Detection System", ICASSP 2020 - 2020 IEEE INTERNATIONAL CONFERENCE ON ACOUSTICS, SPEECH AND SIGNAL PROCESSING (ICASSP), IEEE, 4 May 2020 (2020-05-04), pages 1723-1727, XP033794405, DOI: 10.1109/ICASSP40776.2020.9054753

## Description

### FIELD OF THE INVENTION

The invention is directed to a fall-classification device, to a fall-classification arrangement, to corresponding methods for operating a fall-classification device and a fall-classification arrangement and to a computer program.

### BACKGROUND OF THE INVENTION

US 2020/0163590 A1 discloses a fall detection method, device, and system for detecting whether a target object falls in a detection area. The fall detection method includes: receiving a WIFI signal transmitted by a transmitter in the detection area and extracting CSI data from the WIFI signal, preprocessing the CSI data to obtain CSI data to be identified, and processing the CSI data to be identified through a deep neural network to determine whether the target object falls in the detection area. A prior art fall classification device is also disclosed by US 2019/175074 A1.

### SUMMARY OF THE INVENTION

It would be beneficial to improve an accuracy of a classification of a fall-event type fall detection.

According to the first aspect of the present invention, a fall-classification device is provided. The fall-classification device comprises a receiver unit configured to receive wireless radiofrequency communication signals from one or more external wireless transmitters. Each of the wireless transmitters defines, together with the receiver unit, a respective sensing volume for sensing a plurality of subject-activity metrics. The subject-activity metrics are indicative of a respective subject activity of one or more subjects located within the respective sensing volume.

The fall-classification device also comprises a signal quality determination unit connected to the receiver unit and configured to determine and provide a respective signal-quality value sequence. This sequence includes signal-quality values that are obtained at different points in time. The signal-quality values are indicative of a signal strength of the received wireless communication signal, e.g., a received signal strength indicator (RSSI) or are indicative of a channel state of a wireless communication link between a respective one of the external wireless transmitters and the receiver unit, e.g., a channel state indicator (CSI) or are indicative of any other suitable signal-quality metric that is correlatable to a subject-activity.

Additionally, the fall-classification device comprises a subject-activity data determination unit that is configured to determine and provide, using the provided signal-quality value sequence, respective subject-activity data indicative of at least two subject-activity metrics of the subject within the sensing volume.

The fall classification device further comprises a fall-event detection unit that is connected to the data determination unit and configured to receive the subject-activity data and, based on a time variation of the subject-activity data of at least one subject-activity metric, to determine whether a fall-event of the subject within the sensing volume has occurred and to provide a fall-event signal indicative thereof.

The fall-classification device also comprises a fall-event type classification unit that is connected to the fall event-detection unit and to the subject-activity data determination unit and is configured, upon reception of the fall-event-signal, to determine, using a predetermined algorithm, a type of fall from a predetermined set of detectable types of fall using the subj ect-activity data of at least two subj ect-activity metrics determined during a first time span before the determination of the fall-event and during a second time span after the determination of the fall-event, and to provide a fall-type signal indicative thereof.

The receiver unit is therefore configured to receive wireless radiofrequency communication signals from one or more external wireless transmitters. Between each of the wireless transmitters and the receiving unit a communication link is built. The signals are received at the receiving unit with a signal strength that depends on the transmission power with which the wireless transmitter provides the wireless communication signal and on the location of the wireless transmitter relative to the emitter unit. The signal-quality determination unit is thus configured to determine the respective signal-quality value sequence associated a wireless transmitter. The signal-quality values of a given sequence are indicative of a radio signal strength indicator (RSSI) of the wireless radiofrequency signal received, or of a channel state indicator (CSI) of a wireless communication link between the wireless transmitter and the receiver unit or of any other suitable signal quality metric associated to the wireless radiofrequency communication signals or to the wireless communication link that is correlatable to a given subject-activity, or in other words, of any value obtainable from an analysis of the wireless communication signal or of the wireless communication link that is influenceable by an activity of the subject within the sensing volume.

The fall-classification device is therefore advantageously configured to determine subject-activity data of at least two different subject-activity metrics using the signal quality value sequence associated to a respective one of the external wireless transmitters. The subject-activity data of the corresponding subject-activity metric are those values, either numerical, or Boolean, vectors, or of any other suitable kind, that are inferred from the signal-quality value sequence.

The fall-event detection unit is advantageously configured to determine whether a fall-event of the subject within the sensing volume has occurred and to provide a fall-event signal indicative thereof. The determination of a fall-event is based on a time variation of the subject-activity data of at least one of the subject-activity metric.

The fall-event signal is provided to a fall-event type classification unit, which is configured to determine the type of fall from a predetermined set of types of falls. This is performed by analyzing the subject-activity data of at least two subject-activity metrics determined during a first time span before the determination of the fall-event and during a second time span after the determination of the fall-event. The combined use of the subject-activity data of at least two different subject-activity metrics obtained before and after the determination of the fall-event helps to improve the accuracy of the determination of the fall-event type.

In the following, embodiments of the fall-classification device of the first aspect of the invention will be described.

In an embodiment, the subject-activity metrics that can be sensed by the fall-classification device include subject-activity metrics indicative of a presence or movement of subjects within the sensing volume, such as for instance, location or location coordinate or change in location; distance; displacement; positional characteristics, characteristics associated with movement (e.g. change in position/location) of the subject; lineal or rotational speed; lineal or rotational acceleration; motion direction or angle; azimuth; rotation; path; deformation or transformation such as a shrinking or an expansion, etc. Additionally or alternatively, the subject-activity metrics are indicative of gestures of the subject, including, for example, gait, gait cycle, walking rate; motion of head, hand, mouth chest, eye or other body part, etc. Additionally or alternatively, the subject-activity metrics are indicative of vital signs of the subject and include, for instance, heart-beat rate, breathing rate, interval or variability of heart or breathing rate, tidal volume, depth of breath, inhale time, exhale time, inhale time to exhale time ratio.

Exemplarily, a person's risk of fall can be assessed by determining subject-activity data that is indicative of small balance issues of the subject, wherein abnormal behaviors are considered leading indicators to major fall events. For instance, variability of walking speed compared to the usual baseline of the person is a leading indicator for falls. Similarly, the speed of the person changing from sitting- to standing-position and vice versa is correlatable to a possible fall event.

In an embodiment, the fall-event type classification unit is configured to determine the type of fall by using subject-activity data indicative of the moving speed of a person after a suspected (near-) fall event. After a fall, it is suspected that the person, in particular an elderly person, moves with a less clear direction and slower speed as the person is disoriented.

In an embodiment, the predetermined set of detectable types of falls includes fall-events that can be differentiated from each other in terms of, for example direction of fall (e.g., forward fall, backwards fall, side fall, fall on the spot, etc.), speed of fall (fast fall, slow fall, etc.) or medical urgency (none, low, medium, high, etc.). The detectable types of fall depend on the subject-activity metric that is determined. For instance, the medical urgency of a given fall is best assessed using subject-activity metrics indicative of vital signals. Moreover, the accuracy determination of a given subject-activity metric depends for instance on the frequency band used for the transmission of the wireless radiofrequency communication signals. For instance, breathing detection is more accurate and can also be performed while the subject is moving, and not only in a static position, when high bandwidth technologies such as 60 GHz Wi-Fi are used.

A non-exhaustive list of relevant types of fall that can be determined by an embodiment of the fall-classification device, include:
a) Trip and fall: this is a fast fall from the walking position to the ground.
b) Fall when entering a chair and exiting a chair (static chair or wheelchair or).

Hence, this is a fast fall from the sitting position to the ground.
c) Soft fall: person manages to grab furniture to (somewhat) slow the fall to the floor.
d) Brain stroke fall: slow fall from standing position first to one knee and subsequently down to the ground.
e) Picking-something-up-from-the-floor fall: very prolonged fall (up to two minutes) occurring when the subject, typically an elderly person, tries to pick something up from the floor and does not have the power to get up again and eventually slowly falls to the ground.

Depending on the type of fall that has been identified, the fall-type signal can be provided, preferably via a communication network, for example to a medical or care institution, in order to determine if assistance is needed, what kind of assistance is required and to provide the determined assistance to the person that has fallen. In an embodiment, the fall-classification device is advantageously configured to determine, using the fall-type signal whether an assistance , e.g. medical assistance, is required for the person that has fallen and to provide an assistance-required signal indicative thereof to a medical or care institution.

Alternative embodiments are configured to determine a type of fall from a combination of possible identifiable types of fall formed by a combination of any two or more of the types of fall listed above.

In an embodiment, the receiver unit is configured to receive wireless radiofrequency communication signals in different frequency bands and in accordance with different wireless communication protocols, including, but not limited to, Zigbee, Bluetooth, Bluetooth Low Energy, Wi-Fi, LoRa, etc.

In a particular embodiment, the fall-event detection unit comprises a storage unit comprising fall-data that associates predetermined time variations of the subject-activity data of the respective subject-activity metrics with an expected occurrence of a fall event. In this embodiment, the fall-event detection unit is configured to determine whether the fall-event has occurred using the stored fall data.

In another embodiment, the fall-event type classification unit additionally or alternatively comprises a storage unit that includes fall-type data that associates predetermined combinations of time variations of two or more subject-activity data with a respective fall-type. In this embodiment, the fall-event classification unit is configured to determine the type of fall using the stored fall-type data.

The determination of the type of fall-event might be compromised if the location of the person after the fall does not allow for sufficient sensing quality. This could be due to the person no longer being within the sensing volume, the person presenting a smaller radiofrequency-sensing target once on the floor, the determined signal-quality values being less reliable at certain heights e.g. due to multipath behavior in the room, or if a person is surrounded by other people after the fall.

In a particular embodiment, the fall-event detection unit is configured, upon determination of the fall event, to provide a transmission-adjustment request to the external wireless transmitters which thus operate as wireless transceivers for receiving the transmission-adjustment request. This provision is done either directly, e.g. from the fall-event detection unit over a wireless network to the external wireless transceiver, or indirectly via a routing device such as a hub, a bridge, a router or any other network control node. The transmission-adjustment request is indicative of a request to the external wireless transmitters for providing the wireless communication signals with an increased transmission power. This enables a determination of the value of the signal quality metric, e.g. RSSI, CSI, with a higher resolution, which in turn also enables an increase in the resolution of the determination of the subject-activity data, e.g. location, direction of movement, acceleration, breath rate, heart-beat rate, gestures, etc. The increase of the transmission power is also useful in cases where inter-floor or inter-room sensing is required or used.

Alternatively, or additionally, the transmission-adjustment request is indicative of a request to the external wireless transmitters to alter a transmission beam for transmitting the wireless communication signals. The adaptation or alteration of the beam shape can be for instance a narrowing of the beam or a change in a main direction of the transmission, or a combination thereof. The adaptation of the shape of the transmission beam allows for a confinement of the sensing volume, for example by narrowing the beam, and hence makes the sensing of the wireless communication signals provided by the transmitter with a narrower beam less prone to interferences from objects adjacent to the sensing volume. For instance, breathing detection may be affected by major motion of a ceiling paddle fan.

Additionally, or alternatively, the transmission-adjustment request is indicative of a request to increase a number of external wireless transmitters that provide wireless communication signals for the determination of the signal-quality values. This results in a densification of the number of the wireless transmitters assigned for sensing purposes in the area where the fall-event has occurred, which in turns increases the sensitivity of the determination of the signal-quality values.

Additionally, or alternatively, the transmission-adjustment request is indicative of a request to receive wireless communication signals from a predetermined set of external wireless transmitters, and optionally also according to a predetermined wireless communication protocol. This is advantageous since it enables a change in the sensing topology depending on the location in which the fall-event has occurred. For instance, the transmission-adjustment request is indicative of a request to use a set of wireless transmitters that are closer to the ground or floor.

In another embodiment, the fall-event detection unit is additionally or alternatively configured, upon determination of the fall event, to provide to the receiver unit a reception-adjustment request, indicative of a request to modify a reception volume for receiving the wireless communication signals. The reception volume is indicative of that region of space where signals are receivable with a required signal-quality, such as RSSI or CSI. Thus, in this embodiment, beam shaping is performed additionally, or alternatively, at the receiver unit.

In another embodiment, which can include any of the technical features disclosed above, the subject-activity data determination unit is configured to be operable using at least a high sensitivity grade and a low sensitivity grade for determining the subject-activity data. In this particular embodiment,-upon determination of a fall-event, the fall-event detection unit is configured to provide to the subject-activity data determination unit a sensitivity-increase request signal. In this embodiment, the subject-activity data determination unit, upon reception of the sensitivity-increase request signal, is configured to increase the sensitivity from the low sensitivity grade to the high sensitivity grade for determining the subject-activity data during the second time span. For example, in an embodiment, the subject-activity data determination unit configured to determine and provide, using the provided signal-quality value sequence, respective subject-activity data indicative of at least a vital sign metric, such as breathing or heart-beat rate of the subject within the sensing volume with different sensitivity grades. After determination of the fall event, the subject-activity data determination unit is configured to increase the sensitivity grade from a low sensitivity grade to a high sensitivity grade to determine the subject-activity value, i.e., the value of the vital sign with more accuracy to better assess the type of fall, in particular in view of the medical urgency.

In another embodiment, the fall-event detection unit is additionally or alternatively configured, upon determination of a fall-event, to provide to the subject-activity data determination unit a metric-addition request signal that is indicative of a request to determine subject-activity data of an additional subject-activity metric, and wherein the fall-event-type determination unit is configured to determine the type of fall additionally using the subject-parameter values of the additional subject-parameter. The activity-data determination unit is advantageously configured to determine subject-activity data pertaining to at least three subject-activity metrics. In an exemplary embodiment, the additional subject activity metric is, for example, hand-gestures.

In yet another embodiment, the fall-event detection unit is additionally or alternatively configured to determine a fall-event time span indicative of a duration of the fall-event of the subject within the sensing volume and to provide a fall-duration signal indicative thereof, and wherein the fall-event type classification unit is further configured to determine the type of fall further using the provided fall-duration signal. This embodiment is particularly advantageous in scenarios where it is desired to gather further insights on how the fall event progressed in detail, rather than just looking into the before and after of the occurrence and inferring what happened in the moment of the actual fall-event. For instance, classifying the fall-event time span (e.g. less than 1 second for a fast fall-event, more than 10 seconds for a slow fall) provides background into the severity of the fall-event or even might help to determine whether the fall-event was planned or accidental. The duration of the fall may also consist of duration of each of multiple fall segments as in the case with the brain stroke fall. Thus in an embodiment, the determined fall-event time span is indicative of a respective duration of each of the fall segments.

In yet another embodiment, the fall-event detection unit is additionally or alternatively configured to determine a fall-event context indicative of a fall vertical distance, or of a fall end height, or of a maximum fall speed, of the fall-event of the subject within the sensing volume and to provide a fall-context signal indicative thereof. The fall-event type classification unit is further configured to determine the type of fall further using the provided fall-context signal. This embodiment is also particularly advantageous in scenarios where it is desired to gather further insights on how the fall event progressed in detail, rather than just looking into the before and after of the occurrence and inferring what happened in the moment of the actual fall-event. The subject-activity data determination unit is, for example, configured to determine and monitor the relative height of the breathing/heart movement above the floor level. Hence, if it is determined using for example the fall end height, that the vital sign is currently originating from within 20cm of the ground level, this is indicative of a fall to the ground as the person is sensed to be lying on the floor. If the height of the breathing movement is, for instance, around 50-80 cm it can be indicative of a person on a knee (brainstroke fall as described above as fall type d). Also, for recognizing a soft fall onto furniture, the height of the breathing movement is useful. Also, subject-activity data indicative of a fall vertical distance, or a maximum speed fall can be used to differentiate a fall from a chair or a bed from a fall from a standing position.

In another embodiment, which can also include any of the technical features described above, the fall-event detection unit is additionally or alternatively configured to ascertain context-data indicative of a current context of the sensing volume, and to determine and/or classify the fall-event further in dependence on predetermined associations between different contexts of the sensing volumes with an expected occurrence of the fall event or a type of fall-event. In addition to the subject-activity metrics monitored by the fall-classification device, said device can further rely on other sources of context information that may increase the likelihood that an event is indeed a fall-event. The context data is, in an embodiment, determined by a context-data determination unit. In another embodiment, the context-data is additionally or alternatively received from an external context-data provision device. Suitable context data may include information on a room or area type. Areas more prone to spills or wet surfaces are more likely locations for people to fall. For example, bathrooms, toilets, kitchens, machinery rooms, fountains, outdoor areas equipped with slippery tiles, isles in supermarkets where liquids are stacked, etc. Another type of context-data includes information pertaining to a type of action taking place in the area before/during the fall event. These can further increase a likelihood of a fall-event being (bathroom visit before or after a shower), or simple make any are potentially more hazardous (cleaning crew having just finished wiping a certain area). Yet another kind of ascertainable context data is formed by basic information such as the time of the day (a person may pay less attention when going to the bathroom at night), environmental conditions (amount of light, rainy conditions etc.) or presence of sources of distraction (pets, kids, etc.).

In another embodiment, the fall-event detection unit is further configured to ascertain context-data indicative of a current context of the sensing volume and to determine, using the fall-type signal and the context-data whether an assistance is required and to provide an assistance-required signal indicative thereof. The context data is, in an embodiment, determined by a context-data determination unit. In another embodiment, the context-data is additionally or alternatively received from an external context-data provision device.

In a preferred embodiment, the context-data additionally or alternatively comprises data indicative of a number of people that are currently present in the sensing volume or other specific volume. If multiple persons are present, for instance, in the same room or part of the house, the fall classification device is configured to assume that if a person falls, another person close to that that has fallen will recognize the fall and help, and thus may, under predetermined circumstances, refrain from providing an assistance-request signal. This assistance-request signal can be advantageously provided to an external monitoring team, such as a medical or care center, for a better assessment of the assistance that might be required. More preferably, the context-data also comprises data indicative of a sleep state or wake state of the people in the sensing volume. In an embodiment, fall detection or provision of the assistance-request signal is disabled whenever other people are present in the sensing volume, more preferably only if the other person is awake.

In another embodiment, the context-data additionally or alternatively comprises data indicative of an age of the people within the sensing volume. In an embodiment, the age or age range is determined using ascertained information pertaining to gait speed and/or breathing rate, for instance as monitored by passive WiFi sensing. The context-data may, in an embodiment, additionally or alternatively include user data obtained by geofencing data from the mobile phones carried by the people currently in or close to the sensing volume. This typically requires, in contrast to RF sensing, an active opt-in step of each of the visitors or people in the sensing volume.

Gait speed is considered as one of the most consistent age-associated characteristics for distinguishing whether a person is older than 65 years. In an embodiment, the context-data determination unit, which can be an internal unit of the fall-classification device or an external device or a unit of an external device that is different than the fall-classification device, or the subject-activity data determination unit, is configured to generate a basic motion trail of the people that are in the sensing volume, for example by clocking the time it takes to get from a first location or room to a second location or room. Using the timestamps associated with a predefined motion trail between two locations, the context-data determination unit is configured to estimate the walking speed. Optionally, the context-data determination unit may also determine or estimate the relative height of the physical breathing/heart movement above the floor level and thereby infer the presence/age category of a visitor. Additionally, as children have a noticeably higher resting breathing rate (even with a pronounced age-dependence also the breathing rate determined for example by the subject-activity data determination unit or by any other RF sensing system can be additionally used to infer the age of the visitor.

Thresholds of the fall detection algorithm or the determination of whether (urgent) assistance is required can be fine-tuned depending on whether a high-vitality person beyond the elderly is currently present in the same house or sensing volume.

In an embodiment, the gait and walking pace as subject-activity metrics, are preferably monitored or determined using a first wireless transceiver (transmitter) and a second wireless transceiver (receiver) located in the front & back of the to be monitored person arranged taking into account a sufficiently wide sensing field of view (FOV). For example, while the gait and walking pace monitoring can be done by merely sending, for instance, 30 WiFi sensing messages (wireless radiofrequency communication signals) per second, fall detection requires a higher number (e.g. 1500) of WiFi sensing messages per second. Gait-detection, when using a single WiFi sensing pair of wireless transmitters, requires a wide WiFi sensing FOV to capture a large number of steps. On the other hand, WiFi fall detection only requires that the to-be-monitored person is at any given moment within the (even narrow) FOV of at least one sensing pair of wireless transmitters; hence, unlike for gait detection, for fall detection it is just advantageous to frequently change the node assignment, i.e. the pair of wireless transmitter, for the fall-detection task as the person walks from one sub-space of the room to another.

In an embodiment, the associations between predetermined time variations of the subject activity data with an expected occurrence of a fall event are tuned based on the vitality of the persons or even animals, such as a big-sized dog, that are present in the room. This enables a real-time tuning or adaptations of, e.g., the thresholds of a predetermined fall detection algorithm. This information can be advantageously used to assign which wireless transmitters are to perform the RF sensing, based on which communication link the significant signal-quality value sequence is obtained. For instance, if a high-vitality younger person or big-sized dog is present in the room, the fall detection algorithm may include additional verification steps before reporting that a fall has happened by providing for example the assistance-required signal, and if the fall has happened, that the fall involved the elderly person and not the young visitor jumping from the bed. For instance, the additional steps may include monitoring whether the person lies after the suspected fall for some time on the floor and/or whether the breathing rate has changed after the fall indicating significant stress, as stated above. If a younger person is both present and awake in the same room as the elderly person, the fall detection function may be disabled while the fall detection function may stay always active if a dog is present, as the dog is of no help to the elderly after a fall.

Optionally, an improved algorithm for determining the type of fall may also use information pertaining to the subject's pre-fall moving speed in m/s. A slower speed than a usual speed of for instance 1.5 m/s moving speed is indicative of the elderly person having balance issues making a fall more likely. Balance issues are indicative for most types of fall with obviously the exception of trip-and-fall events. In an embodiment, the moving speed of a person after a suspected (near) fall event is also monitored or determined. After a fall, it is suspected that the elderly person moves with a less clear direction and slower speed due to the shock and discomfort, while a kid while he or she may have indeed fallen to the floor will typically get up back on its feet faster and continue with his or her movement. Hence, the motion statistics of these two events are distinguishable different, allowing the fall-classification device to refine the fall detection. Similarly, a subject-activity metric indicative of motion statistics can also be advantageously employed to distinguish between false and real fall-events such as for instance a person performing a workout routine and a fall-to-the-floor event of the elderly.

In a specific embodiment that is advantageously configured to enable the simultaneous motion-trail/gait detection/fall detection functionality, the external wireless transmitters, e.g., a single wireless transmitter and a first wireless receiver are placed in same plane as the torso of the subject and whereas a second wireless receiver is located at the ceiling. This allows the first sensing pair, i.e., the transmitter and the first receiver, to provide the wireless RF communication signals for monitoring or determining acceleration/deceleration of the torso as a subject-activity metric and the second sensing pair, i.e., the transmitter and the second receiver provide the wireless RF communication signals for monitoring or determining the motion trails/walking gait utilizing wide sensing FOV, for example to distinguish -based on the motion-statics and speed- between the young visitor and the elderly.

As mentioned in the introduction, an important fall-type occurs when the elderly person tries to pick something up from the floor and does not have the power to get up again and eventually slowly falls to the ground. Similarly, another type of fall involves the elderly person feeling dizzy and bending over onto a table or furniture to get support before eventually falling to the floor. As these fall types are progressing very slowly (up to two minutes duration), the inventors recognize that they are very hard to detect with RF sensing. For instance, a prior-art RF sensing system may utilize localization of the breathing movement (i.e. chest height with respect to the floor) to deduce whether a person is standing or lying on the floor. In the case of the prior art systems, if a "pick-something-up-from-the-floor" fall occurs, the system may correctly localize that the breathing occurs at 1m height but based on it and falsely conclude that the 1.2m tall grandchild is present in the room and hence decides to deactivate the fall detection.

To identify and classify more reliably the "slow fall" types with the fall-classification device of the first aspect of the invention, in one embodiment, the fall-event detection unit, is configured to monitor the distance/spatial orientation between wireless external transmitter or transmitters involved in the determination of the signal-quality values and the "mass" that shows the breathing motion (i.e. the rib cage of the person). In this embodiment, the fall-event detection unit is configured to ascertain, using prior motion/activity tracking that only the elderly person is right now present in the space; hence, upon detecting a breathing movement at a predetermined height lower than an expected height, e.g., 1m height, it concludes that the elderly person has been bending over for longer than a given time span, e.g. 1 minute, and raises an alarm in the form of a assistance-required signal. In a related embodiment, instead of localizing the breathing chest movement, a 60GHz RF sensing system in the form of a fall-classification arrangement that receives wireless communication signals from wireless transmitters that communicate in the 60GHz frequency range, is advantageously configured to scan the outline of the body to locate the maximum height of the person with respect to the floor. Combined with the context awareness that the elderly person is present, the s fall-event detection unit can identify an abnormal body position of the elderly indicative of a slow fall.

Subject-activity metrics such as walk metrics that may, for instance, indicate that the (elderly) person is pulling one leg behind when walking are useful for predicting risk of fall. In this case, using mm-wave-based passive RF sensing for extracting walk metrics is a challenge due to the high spatial confinement of mm-wave radio waves in particular due to the narrow field of view of 60GHz WiFi,. Hence, while the RF sensing system may utilize mm-wave RF sensing for fall detection, it may employ other techniques such as 2.4GHz-based WiFi sensing for the gait detection required to determine whether a high-vitality young person is currently present with the elderly.

Alternatively, if only mm-wave wave RF sensing has to be also applied for walking speed/gait detection (e.g. due to WiFi constraints), several pairs of wireless transmitters along the walking path may collaborate to determine the walk metrics over at least a predetermined amount of walking distance, such as, for instance, 5 meters.

A person may be walking down an office corridor containing many wireless transceivers capable whose wireless communication signals can be used for determining the signal quality and thus for RF sensing. In this case, it is advantageous for capturing walk metrics that -while the person walks down the corridor- to first change the sensing field of view of the wireless transmitter and/or the field of view of the wireless receiver. Preferably the pair of wireless transceivers supplying wireless communication signals for RF sensing is chosen such that the RF sensing field of view, typically elongated, is aligned with the current spatial walking-direction of the person. This ensures that the same pair of wireless transceivers is able to follow for a longer time the walking steps of a person for instance for at least five steps (before the fall-classification device has to re-assign the wireless transmitters to a second transmitter-receiver pair of wireless transceivers further down the corridor). In the case of a corridor in an elderly home, or an elongated room, it may be advantageous to utilize a wireless transmitter installed at the ceiling a wireless receiver at a lower height level. This particular spatial combination of a transmitter and receiver, creates an RF sensing volume in the form of a slanted banana. This sensing volume ensures that a first person can be followed over a sufficiently long distance to allow for gait detection (even if a second person walks 2m behind the first person in the same corridor). In particular, if such sensing volume having an approximate shape of the slanted banana is assigned for gait detection, a relatively low number of WiFi messages, such as 30 messages per second, per communication link between transmitter and receiver typically suffices. On the other hand, for effective WiFi fall detection, a larger number of WiFi messages are requires, e.g. 1000 - 1500 WiFi messages per second, and it is advantageous to utilize a pair of wireless transceivers that are placed facing each other from the opposite sides of the corridor. The short distance between the two wireless transceivers and their perpendicular orientation to the motion trail make them well suited to provide wireless communication signals whose signal-quality value is suitable for fall detection.

In another embodiment, the context-data is additionally or alternatively indicative of the physical capabilities of one or more of the subjects present within the sensing volume or close to it. The context-data is for instance indicative of sensorial dysfunction, such as a degree of deafness or blindness, or of mobility impediments, or of any other physical or sensorial limitation that may influence the ability of that person to request medical assistance in case another subject has fallen within the sensing volume. As explained above, in an embodiment, the provision of the assistance-request signal does not take case if the fall-classification device is aware, using the context-data, that another adult that is currently awake is close to the person that has fallen. However, in this preferred embodiment, the fall-classification device is configured to issue the assistance-request signal if the device is aware that the other person close to the person that has fallen has a hearing impediment. Also, as an example, the fall-classification device can be configured to issue the assistance-request signal if the device is aware that another person is in the vicinity of the person that has fallen but he or she is unable to reach the location of the fall event to check on the fallen due to a mobility impediment, for example if the person has fallen in another floor of the house.

According to a second aspect of the present invention, a fall-classification arrangement is provided. The fall-classification arrangement comprises at least one fall-classification device in accordance with the first aspect of the present invention and one or more wireless transmitters configured to provide the wireless communication signals to the at least one fall-classification device.

Thus, the fall-classification arrangement of the invention shares the advantages of the fall-classification device of the first aspect.

In a preferred embodiment, the at least one fall-classification device and the one or more wireless transmitters are network nodes of a wireless communication network, in particular of a wirelessly controlled electrical arrangement, such as a lighting arrangement. In this particular embodiment, the lighting devices are controlled wirelessly and the communication signals used for communication with other network nodes are additionally used for the determination of the signal-quality value. The lighting arrangement may also comprise other network nodes that are not lighting devices, such as switches, sensors, sockets, etc. but which are also configured to receive and provide wireless communication signals that are received by the receiving unit of the fall-classification device. The receiving unit of the fall-classification device may also receive wireless radiofrequency signals from network nodes that do not belong to the same wireless communication network.

According to a third aspect of the present invention, a method for operating a fall-classification device is provided. The method comprises:
- receiving, at a receiver unit, wireless radiofrequency communication signals from one or more external wireless transmitters that, together with the receiver unit, define a respective sensing volume for sensing a plurality of subject-activity metrics [presence, motion, acceleration, gestures, gait, breathing rate, heart rate] indicative of a respective subject activity of one or more subjects located within the sensing volume;
- determining and providing a respective signal-quality value sequence including signal-quality values obtained at different points in time, the signal-quality values being indicative of a signal strength of the received wireless communication signal or being indicative of a channel state of a wireless communication link between a respective one of the external wireless transmitter and the receiver unit;
- determining and providing , using the provided signal-quality value sequence, respective subject-activity data indicative of at least two subject-activity metrics of the subject within the sensing volume;
- based on a time variation of at least one of the at least two subject-activity metrics, determining whether a fall-event of the subject within the sensing volume has occurred and providing a fall-event signal indicative thereof; and
- upon reception of the fall-event-signal, determining, using a predetermined algorithm, a type of fall from a predetermined set of detectable types of fall using the subject-activity values of at least two subject-activity metrics determined during a first time span before the determination of the fall-event and during a second time span after the determination of the fall-event, and to provide a fall-type signal indicative thereof.

The method of the third aspect thus shares the advantages of the fall-classification device of the first aspect of the invention.

In a particular embodiment, the method further comprises, upon determination of the fall event, providing a transmission-adjustment request, indicative of:
- a request to the external wireless transmitters for providing the wireless communication signals with an increased transmission power; or
- a request to increase a number of external wireless transmitters that provide wireless communication signals for the determination of the signal-quality values; or
- a request to receive wireless communication signals from a predetermined set of external wireless transmitters; or any combination thereof:

In yet another embodiment, the method alternatively or additionally comprises the step of providing, upon determination of a fall-event, a metric-addition request signal that is indicative of a request to determine subject-act values of an additional subject-parameter, and the step of determining the type of fall additionally using the subject-parameter values of the additional subject-parameter.

In another embodiment, the method additionally or alternatively comprises the steps of:
- determining a fall-event time span indicative of a duration of the fall-event of the subject within the sensing volume and providing a fall-duration signal indicative thereof;
- determining the type of fall further using the provided fall-duration signal.

According to a fourth aspect of the present invention, a method for operating a fall-classification arrangement is provided. The method comprises:
- providing wireless communication signals; and
- performing the method of the third aspect of the invention.

The method of the fourth aspect thus shares the advantages of the fall-classification arrangement of the second aspect of the invention.

A fifth aspect of the present invention is provided by a computer program comprising instructions that, when executed by a computer, cause the computer to carry out the methods of any of the claims 12 to 14.

A sixth aspect of the present invention is provided by a fall-determination device that comprises a receiver unit configured to receive wireless radiofrequency communication signals from one or more external wireless transmitters which, together with the receiver unit, define a respective sensing volume for sensing one or more subjects located within the sensing volume. The fall-determination device also comprises a signal quality determination unit that is connected to the receiver unit and that is configured to determine and provide a respective signal-quality value sequence including signal-quality values obtained at different points in time, the signal-quality values being indicative of a signal strength of the received wireless communication signal or being indicative of a channel state of a wireless communication link between a respective one of the external wireless transmitter and the receiver unit or being indicative of any other suitable signal-quality metric that is correlatable to fall movement of the subject. The fall-determination device also comprises a fall-event detection unit that is connected to the signal quality determination unit and configured to receive the signal-quality value sequence therefrom. The fall-event determination unit is further configured to ascertain context-data indicative of a current context of the sensing volume and/or of the subjects located within the sensing volume and to determine, using the received signal-quality value sequence and the ascertained context-data whether a fall of a subject in the sensing volume has occurred.

The fall-determination device of the sixth aspect of the present invention enables the reduction of false alarms in fall detection, near-fall detection and risk-of-fall detection systems, for example, but not limited to, in elderly care, by using ascertained context data indicative of a current context of the sensing volume, which may also include context data indicative of characteristics of the subjects present in the sensing volume, as discussed above, to determine whether a fall-event has occurred. The fall-determination device is advantageously configured to leverage context-awareness about presence and specific characteristics of the subjects in the sensing volume, to fine-tune the operation of the fall detection solution. As a result of the improved fall-determination operation, false fall-detection-triggers when, for instance, the elderly person is visited by or shares the house with a highly physically active family member or large dog, are minimized.

In an embodiment, the fall-event determination unit is configured to ascertain context-data indicative of a respective age range of the subjects based on, for instance, the gait speed and/or breathing rate. Then, the thresholds for determining the occurrence of a fall-event based on the signal-quality value sequence are fine-tuned according to the context-data, depending for instance on whether a second high-vitality person --beyond the elderly-- is currently present in the same house.

In a preferred embodiment, the fall-event detection unit is further configured to determine whether assistance is required for the fallen subject and to provide an assistance-required signal indicative thereof.

The fall-event detection unit is preferably adapted to ascertain any combination of context-data as discussed above.

In a preferred embodiment, the context-data comprises data indicative of a number of people that are currently present in the sensing volume or other specific volume, and also, preferably of any physical dysfunctions, such as sensorial or mobility impediments of the people, as explained above with respect to the fall-classification device of the first aspect of the invention. If multiple persons are present, for instance, in the same room or part of the house, the fall classification device is configured to assume that if a person falls, another person close to that that has fallen will recognize the fall and help, and thus may, under predetermined circumstances, refrain from providing an assistance-request signal. This assistance-request signal can be advantageously provided to an external monitoring team, such as a medical or care center, for a better assessment of the assistance that might be required. More preferably, the context-data also comprises data indicative of a sleep state or wake state of the people in the sensing volume. In an embodiment, fall detection or provision of the assistance-request signal is disabled whenever other people are present in the sensing volume, more preferably only if the other person is awake.

In another embodiment, the context-data also comprises data indicative of an age of the people within the sensing volume. In an embodiment, the age or age range is determined using ascertained information pertaining to gait speed and/or breathing rate, for instance as monitored by passive WiFi sensing. The context-data may, in an embodiment, additionally or alternatively include user data obtained by geofencing data from the mobile phones carried by the people currently in or close to the sensing volume. This typically requires, in contrast to RF sensing, an active opt-in step of each of the visitors or people in the sensing volume.

Gait speed is considered as one of the most consistent age-associated characteristics for distinguishing whether a person is older than 65 years. In an embodiment, the context-data determination unit, which can be an internal unit of the fall-determination device or an external device or a unit of an external device that is different than the fall-determination device, is configured to generate a basic motion trail of the people that are in the sensing volume, for example by clocking the time it takes to get from a first location or room to a second location or room. Using the timestamps associated with a predefined motion trail between two locations, the context-data determination unit is configured to estimate the walking speed. Optionally, the context-data determination unit may also determine or estimate the relative height of the physical breathing/heart movement above the floor level and thereby infer the presence/age category of a visitor. Additionally, as children have a noticeably higher resting breathing rate (even with a pronounced age-dependence also the breathing rate determined for example by a subject-activity data determination unit or by any other RF sensing system can be additionally used to infer the age of the visitor.

Thresholds of the fall detection algorithm or the determination of whether (urgent) assistance is required can be fine-tuned depending on whether a high-vitality person beyond the elderly is currently present in the same house or sensing volume.

In an embodiment, the gait and walking pace as subject-activity metrics, are preferably monitored or determined using a first wireless transceiver (transmitter) and a second wireless transceiver (receiver) located in the front & back of the to be monitored person arranged taking into account a sufficiently wide sensing field of view (FOV). For example, while the gait and walking pace monitoring can be done by merely sending, for instance, 30 WiFi sensing messages (wireless radiofrequency communication signals) per second, fall detection requires a higher number (e.g. 1500) of WiFi sensing messages per second. Gait-detection, when using a single WiFi sensing pair of wireless transmitters, requires a wide WiFi sensing FOV to capture a large number of steps. On the other hand, WiFi fall detection only requires that the to-be-monitored person is at any given moment within the (even narrow) FOV of at least one sensing pair of wireless transmitters; hence, unlike for gait detection, for fall detection it is just advantageous to frequently change the node assignment, i.e. the pair of wireless transmitter, for the fall-detection task as the person walks from one sub-space of the room to another.

In an embodiment, the associations between predetermined time variations of the signal-quality data with an expected occurrence of a fall event are tuned based on the vitality of the persons or even animals, such as a big-sized dog, that are present in the room. This enables a real-time tuning or adaptations of, e.g., the thresholds of a predetermined fall determination algorithm. This information can be advantageously used to assign which wireless transmitters are to perform the RF sensing, based on which communication link the significant signal-quality value sequence is obtained. For instance, if a high-vitality younger person or big-sized dog is present in the room, the fall determination algorithm may include additional verification steps before reporting that a fall has happened by providing for example the assistance-required signal, and if the fall has happened, that the fall involved the elderly person and not the young visitor jumping from the bed. For instance, the additional steps may include monitoring whether the person lies after the suspected fall for some time on the floor and/or whether the breathing rate has changed after the fall indicating significant stress, as stated above. If a younger person is both present and awake in the same room as the elderly person, the fall determination function may be disabled while the fall determination function may stay always active if a dog is present, as the dog is of no help to the elderly after a fall.

Optionally, an improved algorithm for determining an occurrence of a fall may also use information pertaining to the subject's pre-fall moving speed in m/s. A slower speed than a usual speed of for instance 1.5 m/s moving speed is indicative of the elderly person having balance issues making a fall more likely. Balance issues are indicative for most types of fall with obviously the exception of trip-and-fall events. In an embodiment, the moving speed of a person after a suspected (near) fall event is also monitored or determined. After a fall, it is suspected that the elderly person moves with a less clear direction and slower speed due to the shock and discomfort, while a kid while he or she may have indeed fallen to the floor will typically get up back on its feet faster and continue with his or her movement. Hence, the motion statistics of these two events are distinguishable different, allowing the fall-determination device to refine the fall determination operation Similarly, a signal-quality data indicative of motion statistics can also be advantageously employed to distinguish between false and real fall-events such as for instance a person performing a workout routine and a fall-to-the-floor event of the elderly.

In a specific embodiment that is advantageously configured to enable the simultaneous motion-trail/gait detection/fall detection functionality, the external wireless transmitters, e.g., a single wireless transmitter and a first wireless receiver are placed in same plane as the torso of the subject and whereas a second wireless receiver is located at the ceiling. This allows the first sensing pair, i.e., the transmitter and the first receiver, to provide the wireless RF communication signals for monitoring or determining acceleration/deceleration of the torso and the second sensing pair, i.e., the transmitter and the second receiver provide the wireless RF communication signals for monitoring or determining the motion trails/walking gait utilizing wide sensing FOV, for example to distinguish -based on the motion-statics and speed- between the young visitor and the elderly.

As mentioned in the introduction, an important fall-type occurs when the elderly person tries to pick something up from the floor and does not have the power to get up again and eventually slowly falls to the ground. Similarly, another type of fall involves the elderly person feeling dizzy and bending over onto a table or furniture to get support before eventually falling to the floor. As these fall types are progressing very slowly (up to two minutes duration), the inventors recognize that they are very hard to detect with RF sensing. For instance, a prior-art RF sensing system may utilize localization of the breathing movement (i.e. chest height with respect to the floor) to deduce whether a person is standing or lying on the floor. In the case of the prior art systems, if a "pick-something-up-from-the-floor" fall occurs, the system may correctly localize that the breathing occurs at 1m height but based on it and falsely conclude that the 1.2m tall grandchild is present in the room and hence decides to deactivate the fall detection.

To identify more reliably the "slow fall" types with the fall-determination device of the sixth aspect of the invention, in one embodiment, the fall-event detection unit, is configured to monitor the distance/spatial orientation between wireless external transmitter or transmitters involved in the determination of the signal-quality values and the "mass" that shows the breathing motion (i.e. the rib cage of the person). In this embodiment, the fall-event detection unit is configured to ascertain, using prior motion/activity tracking that only the elderly person is right now present in the space; hence, upon detecting a breathing movement at a predetermined height lower than an expected height, e.g., 1m height, it concludes that the elderly person has been bending over for longer than a given time span, e.g. 1 minute, and raises an alarm in the form of a assistance-required signal. In a related embodiment, instead of localizing the breathing chest movement, a 60GHz RF sensing system in the form of a fall-determination arrangement that receives wireless communication signals from wireless transmitters that communicate in the 60GHz frequency range, is advantageously configured to scan the outline of the body to locate the maximum height of the person with respect to the floor. Combined with the context awareness that the elderly person is present, the s fall-event detection unit can identify an abnormal body position of the elderly indicative of a slow fall.

Subject-activity metrics that are inferable from the signal quality data, such as walk metrics, may for instance indicate that the (elderly) person is pulling one leg behind when walking are useful for predicting risk of fall. In this case, using mm-wave-based passive RF sensing for extracting walk metrics is a challenge due to the high spatial confinement of mm-wave radio waves in particular due to the narrow field of view of 60GHz WiFi,. Hence, while the RF sensing system may utilize mm-wave RF sensing for fall determination, it may employ other techniques such as 2.4GHz-based WiFi sensing for the gait detection required to determine whether a high-vitality young person is currently present with the elderly.

Alternatively, if only mm-wave wave RF sensing has to be also applied for walking speed/gait detection (e.g. due to WiFi constraints), several pairs of wireless transmitters along the walking path may collaborate to determine the walk metrics over at least a predetermined amount of walking distance, such as, for instance, 5 meters.

A person may be walking down an office corridor containing many wireless transceivers capable whose wireless communication signals can be used for determining the signal quality and thus for RF sensing. In this case, it is advantageous for capturing walk metrics that -while the person walks down the corridor- to first change the sensing field of view of the wireless transmitter and/or the field of view of the wireless receiver. Preferably the pair of wireless transceivers supplying wireless communication signals for RF sensing is chosen such that the RF sensing field of view, typically elongated, is aligned with the current spatial walking-direction of the person. This ensures that the same pair of wireless transceivers is able to follow for a longer time the walking steps of a person for instance for at least five steps (before the fall-classification device has to re-assign the wireless transmitters to a second transmitter-receiver pair of wireless transceivers further down the corridor). In the case of a corridor in an elderly home, or an elongated room, it may be advantageous to utilize a wireless transmitter installed at the ceiling a wireless receiver at a lower height level. This particular spatial combination of a transmitter and receiver, creates an RF sensing volume in the form of a slanted banana. This sensing volume ensures that a first person can be followed over a sufficiently long distance to allow for gait detection (even if a second person walks 2m behind the first person in the same corridor). In particular, if such sensing volume having an approximate shape of the slanted banana is assigned for gait detection, a relatively low number of WiFi messages, such as 30 messages per second, per communication link between transmitter and receiver typically suffices. On the other hand, for effective WiFi fall determination, a larger number of WiFi messages are requires, e.g. 1000 - 1500 WiFi messages per second, and it is advantageous to utilize a pair of wireless transceivers that are placed facing each other from the opposite sides of the corridor. The short distance between the two wireless transceivers and their perpendicular orientation to the motion trail make them well suited to provide wireless communication signals whose signal-quality value is suitable for fall detection.

Further, different embodiments of the fall-determination device of the sixth aspect include any suitable feature or element of the fall-classification device of the first aspect.

For instance, in an embodiment, the fall-determination device comprises a - a subject-activity data determination unit configured to determine and provide, using the provided signal-quality value sequence, respective subject-activity data indicative of at least one subject-activity metric of the subject within the sensing volume, and the fall-event detection unit is configured to determine, using the received subject-activity data and the ascertained context-data whether a fall of a subject in the sensing volume has occurred.

In another embodiment, the fall-determination device also comprises a fall-event type classification unit connected to the fall event-detection unit and to the subject-activity data determination unit and configured, upon determination of an occurrence of a fall-event, e.g. following a reception of a fall-event signal indicative thereof, to determine, using a predetermined algorithm, a type of fall from a predetermined set of detectable types of fall using the subject-activity data. Preferably, to increase the accuracy of the fall-type selection, the determination is performed using subject activity data of at least two subject-activity metrics determined during a first time span before the determination of the fall-event and during a second time span after the determination of the fall-event, and to provide a fall-type signal indicative thereof.

In another embodiment, the fall-event detection unit comprises a storage unit comprising fall-data that associates predetermined time variations of the signal-quality value sequence of the respective subject-activity metrics with an expected occurrence of a fall event, and wherein the fall-event detection unit is configured to determine whether the fall-event has occurred using the stored fall data.

As in the case of the fall-classification device of the first aspect, in an embodiment of the fall-determination device of the sixth aspect, the fall-event detection unit is configured, upon determination of the fall event, to provide to the external wireless transmitters a transmission-adjustment request, indicative of:
- a request to the external wireless transmitters for providing the wireless communication signals with an increased transmission power and/or to modify a transmission beam for transmitting the wireless communication signals or
- a request to increase a number of external wireless transmitters that provide wireless communication signals for the determination of the signal-quality values; or
- a request to receive wireless communication signals from a predetermined set of external wireless transmitters; or any combination thereof.

The fall-determination device of the sixth aspect of the invention is advantageously configured to be part of a fall-classification arrangement that comprises at least one fall-determination device and one or more wireless transmitters configured to provide the wireless communication signals to the at least one fall-determination device. In particular, the wireless transmitters are preferably network nodes of a wirelessly controllable lighting arrangement.

A seventh aspect of the present invention is formed by a method for operating a fall-determination device. The method comprises the steps of
- receiving wireless radiofrequency communication signals, in particular from one or more external wireless transmitters which, together with a receiver unit of the fall-determination device, define a respective sensing volume for sensing one or more subjects located within the sensing volume;
- determining and providing a respective signal-quality value sequence including signal-quality values obtained at different points in time, the signal-quality values being indicative of a signal strength of the received wireless communication signal or being indicative of a channel state of a wireless communication link between a respective one of the external wireless transmitter and the receiver unit of the fall-determination device or being indicative of any other suitable signal-quality metric that is correlatable to a fall movement of the subject;
- ascertaining context-data indicative of a current context of the sensing volume and/or of the subjects located within the sensing volume; and
- determining, using the received signal-quality value sequence and the ascertained context-data whether a fall of a subject in the sensing volume has occurred.

Further, the invention is directed to a computer program comprising instructions that, when executed by a computer, cause the computer to carry out the methods for controlling operation of a fall-determination device in accordance with the seventh aspect.

It shall be understood that the fall-classification device of claim 1, the fall-classification arrangement of claim 10, the method for operating a fall-classification device of claim 11, the method for operating a fall-classification arrangement of claim 14 and the computer program of claim 15, as well as the fall-determination device, the fall determination arrangement, the method for controlling operation of the fall-determination arrangement and the corresponding computer program, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows a schematic block diagram of an embodiment of a fall-classification arrangement,
Fig. 2 shows a schematic diagram of another exemplary fall-classification arrangement,
Fig. 3 shows a schematic flow diagram of a method for operating a fall-classification device,
Figs. 4A and 4B show a schematic flow diagram of a another embodiment of a method for operating a fall-classification device,
Fig. 5 shows a flow diagram of an embodiment of a method for operating a fall-classification arrangement,
Fig. 6 shows a schematic diagram of an embodiment of a fall-determination device forming part of a fall-determination arrangement, and
Fig. 7 shows a flow diagram of an embodiment of a method for operating a fall-determination device.

### DETAILED DESCRIPTION OF EMBODIMENTS

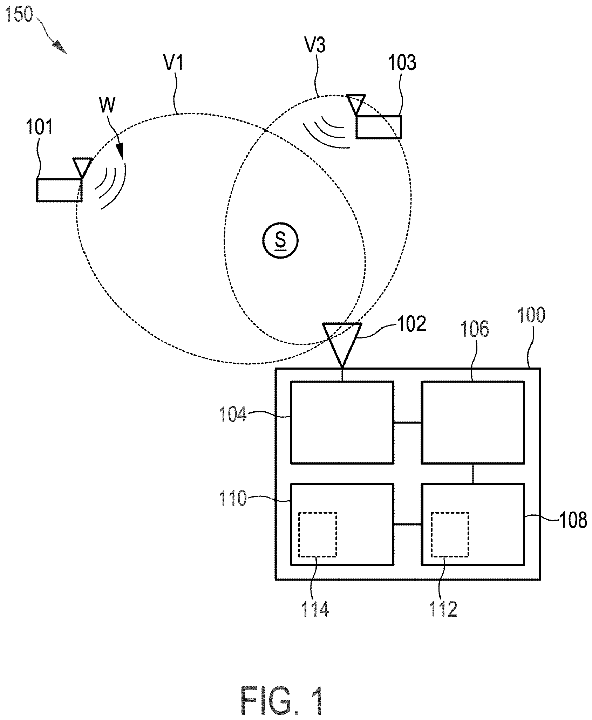

Fig. 1 shows a schematic block diagram of an embodiment of a fall-classification arrangement 150 that comprises a wireless transmitters 101 and 103 and a fall-classification device 100. The wireless transmitters are configured to provide wireless radiofrequency communication signals W according to one or more wireless communication protocols. The fall-classification device 100 comprises a receiver unit 102 that is configured to receive the wireless radiofrequency communication signals W from the wireless transmitters 101, 103. Each of the wireless transmitters 101, 103 define, together with the receiver unit 102, a respective sensing volume V 1, V3 for sensing a plurality of subject-activity metrics indicative of a respective subject activity of one or more subjects S located within the sensing volume. In Figure 1, the geometry of the respective sensing volumes V1, V3 has been simplified for the sake of clarity and have the form of an ellipsoid. Within the sensing volume, the presence of the subject alters the signals that the receiving unit receives from the wireless transmitters, so that changes in the received signal can be associated to activities of the subject. These activities that affect the wireless communication signal in a predictable manner are referred to as subject-activity metrics, and include, for example, location or location coordinate or change in location; distance; displacement; positional characteristics, characteristics associated with movement (e.g. change in position/location) of the subject; lineal or rotational speed; lineal or rotational acceleration; motion direction or angle; azimuth; rotation; path; deformation or transformation such as a shrinking or an expansion, etc. Additionally or alternatively, the subject-activity metrics are indicative of gestures of the subject, including, for example, gait, gait cycle, walking rate; motion of head, hand, mouth chest, eye or other body part, etc. Additionally or alternatively, the subject-activity metrics are indicative of vital signs of the subject and include, for instance, heart-beat rate, breathing rate, interval or variability of heart or breathing rate, tidal volume, depth of breath, inhale time, exhale time, inhale time to exhale time ratio.

The fall-classification device also comprises a signal quality determination unit 104 that is connected to the receiver unit 102 and configured to determine and provide a respective signal-quality value sequence including signal-quality values obtained at different points in time. The signal-quality values are indicative of a signal strength of the received wireless communication signal (RSSI), or are indicative of a channel state of a wireless communication link between a respective one of the wireless transmitters 101, 103 and the receiver unit 102 (CSI) or are in general indicative of any suitable signal-quality metric that is correlatable to a subject-activity, i.e. of any value obtainable from an analysis of the wireless communication signals W or of the wireless communication links, that is influenceable by an activity of the subject S within the sensing volume V1, V3.

The fall-classification device 100 also comprises a subject-activity data determination unit 106 that is configured to determine and provide, using the signal-quality value sequence provided by the signal quality determination unit 104, respective subject-activity data indicative of at least two subject-activity metrics of the subject S within the sensing volume V1, V3. Depending on the subject-activity metric being determine, the values can be Boolean, such as in the case of the subject-activity being indicative of a presence or absence of the subject within the respective sensing volume, numerical, such as in the case of the subject-activity metric being indicative of vital signs such as breathing rate or heart-beat rate or in the case of a position in a 2D or 3D space, vectors in the case of for example speed, acceleration, deceleration, or other type of values indicative of, for example, respective gestures of the subject.

The fall-classification device 100 also comprises a fall-event detection unit 108 that connected to the subject-activity data determination unit 106 and that is configured to receive the subject-activity data and, based on a time variation of the subject-activity data of at least one subject-activity metric, to determine whether a fall-event of the subject S within the sensing volume V1, V3 has occurred. The fall-event detection unit is also configured to provide a fall-event signal indicative of a determination of an occurrence of a fall-event. Optionally, the fall-event detection unit 108 comprises a storage unit 112 comprising fall-data that associates predetermined time variations of the subject-activity data of the respective subject-activity metrics with an expected occurrence of a fall event. In this particular case the fall-event detection unit is configured to determine whether the fall-event has occurred using the stored fall data.

Further, the fall-classification device includes a fall-event type classification unit 110 that is connected to the fall event-detection unit 108 and to the subject-activity data determination unit 106 and that is configured, upon reception of the fall-event-signal, to determine, using a predetermined algorithm, a type of fall from a predetermined set of detectable types of fall using the subject-activity data of at least two subject-activity metrics determined during a first time span before the determination of the fall-event and during a second time span after the determination of the fall-event, and to provide a fall-type signal indicative thereof. Optionally, the fall-event type classification unit 110 comprises a storage unit 114 that includes fall-type data that associates predetermined combinations of time variations of two or more subject-activity data with a respective fall-type, and wherein the fall-event classification unit is configured to determine the type of fall using the stored fall-type data

The predetermined set of detectable types of falls includes fall-events that can be differentiated from each other in terms of, for example direction of fall (e.g., forward fall, backwards fall, side fall, fall on the spot, etc.), speed of fall (fast fall, slow fall, etc.) or medical urgency (none, low, medium, high, etc.). The detectable types of fall depend on the subject-activity metric that is determined. For instance, the medical urgency of a given fall is best assessed using subject-activity metrics indicative of vital signals. Moreover, the accuracy determination of a given subject-activity metric depends for instance on the frequency band used for the transmission of the wireless radiofrequency communication signals. For instance, breathing detection is more accurate and can also be performed while the subject is moving, and not only in a static position, when high bandwidth technologies such as 60 GHz Wi-Fi are used.

In an exemplary fall-classification arrangement, it is proposed to track with RF sensing techniques, as explained above, the presence and the breathing rate of the subject S throughout a space or area of interest, i.e. the sensing volume. This could be for example a nursing home/apartment, whereby the elderly person is monitored via RF sensing deployed via the wireless transmitters. Breathing detection is already possible with current technologies when the subjects are relatively static (e.g. sleeping, sitting down), but higher bandwidth technologies like 60GHz Wi-Fi, as their wireless signals are more confined, enable also to track the breathing rate while there's significant motion (e.g. the subject is moving).

Thus, in this case subject-activity data in the form of a numerical value of the breathing rate of a subject is determined and monitored using the signal-quality value of the wireless communication signals, while the presence of the subject in the room is also monitored. The fall-event detection unit 108 receives the values of the breathing rate. For example, an elderly person in steady state could have a normal breathing rate of 15bpm (breaths per minute). Assuming no significant breathing issues, this level should be roughly constant as the elderly person moves around the house.

If the subject would e.g. fall to the ground, the breathing signal would momentarily be lost due to the fast movements (acceleration/deceleration) during the fall. This time variation of the monitored breathing rate is identified by the fall-event detection unit as being indicative of a fall event, and provides a fall-event signal. Once the person lies still on the floor, the fall-determination device continues tracking the breathing rate and compare it with the pre-fall levels. The analysis of the subject-activity data before the provision of event (e.g. a substantially constant breathing rate plus a sudden gap in the breathing data) and after the event is used to both identify a fall and determine, coarsely, its type. For example:
- If the subject is still detected in the room and the values before and after the gap in data are roughly the same, it could simply mean that there was no real fall but just an issue in detection, blind spot, loss of synchronization with chest movements, etc. Similarly, the person may have lied down on the floor to perform yoga.
- If the subject is still detected in the room and the breathing values after the time gap are somewhat higher, it could mean that the person has suffered a near/soft fall, leading to a moderate increase/irregularity in breathing rate. The fall-classification device system interprets this as indicative of a near accident and keeps track of the statistics without any real time action required.
- If the subject is still detected in the room and the breathing values after the time gap are significantly higher or above certain thresholds, it could mean that the subject has suffered a dangerous situation and as a result is nervous/worried/hurt, which inevitably leads to the increase/irregularity in breathing rate. The sensing system interprets this as indicative of an accident urgently requiring attention.
- If the subject is still detected in the room and the values after the gap are significantly lower or below certain thresholds, it could mean that the subject has suffered an even more dangerous situation that has rendered them unconscious (and therefore less responsive, not psychologically stressed, etc. which would have elevated their breathing rate). This could indicate immediate, highly urgent attention by first responders.

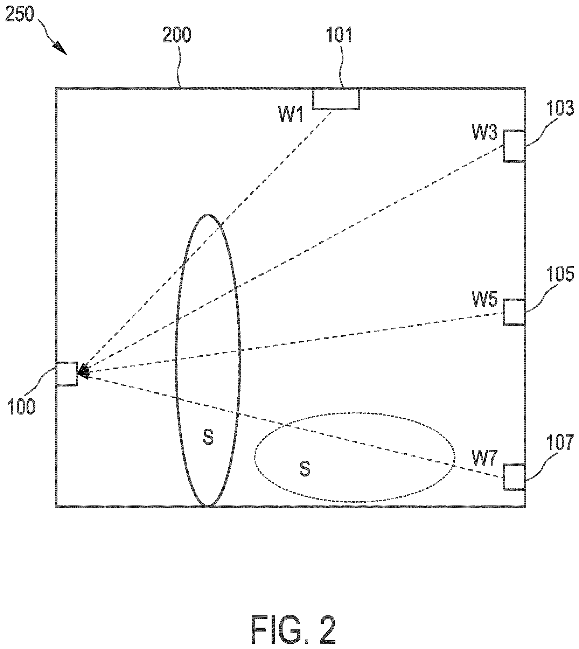

Fig. 2 shows a schematic diagram of another exemplary fall-classification arrangement 250. In this particular example, the wireless transmitters 101, 103, 105 and 107, and the fall-classification device 100 are network nodes of a wireless communication network. In particular, the devices stated above are network nodes of a wirelessly controllable lighting arrangement. For example wireless transmitter 101 is a wirelessly controllable lighting device in the form of a luminaire installed on a ceiling of room 200. Wireless transmitter 103 is for instance an "EXIT" sign that also includes a wirelessly controllable light emitting. Wireless transmitter 105 is a switch configured to wirelessly control the operation of luminaire 101. Wireless transmitter 107 is a socket that is also configured to wirelessly provide use data. The fall-classification device 100 is in this particular example integrated in a wireless communication network control device, such as a hub, a bridge, a router, an access point, a gateway, etc. In other examples, the fall-classification device can be integrated in any type of node of the wireless communication network. A subject S is in the room standing, as indicated by the solid line. The fall-classification device 100 monitors subject-activity metrics associated to the subject using the wireless communication signals provided by the nodes of the fall-detection arrangement 250. At a given moment in time, the person S falls to the floor, as indicated by the discontinuous line. The fall-event is detected by the fall-event determination unit of the device 100, which provides a fall-event signal indicative thereof.

The classification of the fall-event might be compromised if the location of the person after the fall-event does not allow for a received wireless communication signal with sufficient signal quality. This could be due to the person no longer being within the sensing volume, the person presenting a smaller target once in the floor, the wireless communication signal being less reliable at certain heights, e.g. due to multipath behavior in the room, or if a person is surrounded by many other people after the fall, etc.

Under these circumstances, an exemplary fall-classification device is optionally configured to reconfigures itself upon detecting events that could indicate a possible fall (e.g. normal breathing rate followed by lack of breathing information)

In this exemplary fall detection arrangement 250, the fall-event detection unit 108 is configured, upon determination of the fall event, to provide to the external wireless transmitters, directly in a single hop communication or indirectly via a multi-hop communication, a transmission-adjustment request.

The transmission-adjustment request can be, for instance, indicative of a request to one or more of the wireless transmitters 101, 103, 105, 107, for providing the wireless communication signals with an increased transmission power. This increases the sensitivity of the determination of the signal quality values after the fall, which are relevant for the classification of the fall-event into one of the predetermined types of fall.

Additionally, or alternatively, the transmission-adjustment request is, for instance, a request to increase a number of external wireless transmitters that provide wireless communication signals for the determination of the signal-quality values. For example, if before the determination of the fall-event, only devices 101, 103 and 105 were providing the wireless communication signals for the determination of the signal-quality data and, based thereon, of the subject-activity data of the plurality of subject-activity metrics, the transmission-adjustment request can be indicative of a request to device 107 to also provide wireless communication signals for the determination of the subject-activity data of the subject who is now on the floor.

Alternatively, the transmission-adjustment request can be indicative of a request to receive wireless communication signals from a predetermined set of wireless transmitters. This enables a change in the sensing topology, for example from using signals provided by the ceiling lights only to using signals from those transmitters which are expected to be closer to the ground like plugs and light strips, or from single floor sensing to inter-floor sensing in case of this happening on other floors. Inter-floor sensing refers to the use of wireless communication signals provided by wireless transmitters that are located in another floor, typically in the floor below. A person that has fallen on the ground has a larger impact on those signals coming from the ceiling of the floor below than when the person is standing.

In another exemplary fall-classification device, the subject-activity data determination unit 106 is configured to be operable using at least a high sensitivity grade and a low sensitivity grade for determining the subject-activity data. Typically the subject-activity data determination unit operates using the low sensitivity grade, since is uses less resources. In this fall-classification device, and upon determination of a fall-event, the fall-event detection unit 108 is configured to provide to the subject-activity data determination unit 106 a sensitivity-increase request signal. The subject-activity data determination unit 106, upon reception of the sensitivity-increase request signal is configured to increase the sensitivity from the low sensitivity grade to the high sensitivity grade for determining the subject-activity data during the second time span.

In another exemplary fall-classification arrangement, the fall-classification device 100 is optionally configured to use other subject-activity metrics and the related subject-activity data obtained in the before- and after-the-fall-event periods. For example, a fall-classification arrangement installed in a retail store can measure both breathing rate as well keep track of the direction of motion as two monitored subject-activity metrics. This way, falls can be more accurately detected as now multiple factors indicative of the fall can be detected by the fall-classification device (e.g. momentary gap in breathing rate data as well as a lack of major or even minor motion after the fall).

The fall-event detection unit 108 of the fall-classification device 100 is optionally configured, upon determination of a fall-event, to provide to the subject-activity data determination unit 106 a metric-addition request signal that is indicative of a request to determine subject-activity data of an additional subject-activity metric. The fall-event-type determination unit 110 is configured to determine the type of fall additionally using the subject-activity data of the additional subject-activity metric.

Thus, he proposed additional subject-activity metric also enables a further refinement of the fall classification. For instance, the metric-addition request signal is now indicative of a request to determine subject-activity data indicative of a gait of the subject, which is the exemplary additional subject-activity metric. If the values of breathing rate after the fall-event are significantly lower than before the fall, but subject-activity data determination unit still determines the usual gait associated with normal walking without pain after the suspected fall, the suspicious motion activity could simply indicate a false negative on breathing (i.e. there was really no fall).

If the values of breathing rate after the fall are significantly higher than before the fall and the subject-activity data determination unit detects a walk with unusual gait, this could indicate that the person might have fallen but either got up, is crawling in a certain direction, or is walking painfully. The fall-type signal provided in this case can be therefore a request to dispatch an employee to check on consumer but not to call the emergency services, or simply determine that it was a soft, inconsequential fall but that had no major medical impact beyond scaring the customer. On the other hand, increased breathing rate with no/very low motion activity could indicate a person conscious but no longer able to move.

In another exemplary fall-classification device 100 the fall-event detection unit 108 is further configured to determine a fall-event time span indicative of a duration of the fall-event of the subject S within the sensing volume V and to provide a fall-duration signal indicative thereof. In this exemplary fall-classification device 100, the fall-event type classification unit 110 is further configured to determine the type of fall further using the provided fall-duration signal.

For example, a person might simple faint due to e.g., an aneurism, low blood pressure dip, etc. In this situations the subject becomes unconscious and, as a result, the body simply drops, in the sense that the subject cannot do anything to slow or soften the fall. This fall results is a quick fall-event of less than 1 second, sometimes even less than half a second. On the other hand, a person might trip on something and still manage to hold onto something (furniture, objects, etc.) resulting in a soft fall or a near-fall. The suddenly holding onto something is a natural reaction from people to avoid damaging themselves during the fall, and, even though the holding might not prevent the subject ending up on the floor, it might be enough to slow the fall-event and prevent serious injuries.

The subject-activity data determination unit 106 is configured to determine a time series of the position of the torso of the subject, as the torso is the body mass which contributes the most to radiofrequency based detection. Hence, by analyzing how the torso shifts in height and classifying different speeds of the torso, the type of fall can be inferred. For instance, the subject-activity data determination unit 106 determines data that is indicative of the fact that the subject distorting the radiofrequency communication signals W is gradually shrinking, which could be simply because the person is gradually moving towards the floor, or curling up, etc. On the other hand, a sudden variation the body mass interacting with the wireless communication signals W can be indicative of a fast fall event. How fast these transitions have occurred, i.e. how long the fall-event time span is, is an indication of how much the person was able to prevent the fall and thus provides an indication for the classification of the fall-type, in particular in view of the risk of injury.

This particular fall-classification device 100 is advantageously suitable to determine to some certainty whether a fall-event could have been staged or faked. For example, if a goal is to scam an insurance company by claiming to have fallen to the ground, the subject might prefer to comfortably lower himself to the floor rather than abruptly dropping and risking hurting himself for real. This can be enriched by comparing other subject-activity metrics such as vital signs, e.g. breathing rates. A person who accidentally falls might exhibit a clearer difference between the before- and after the fall- breathing rates, as they would have normally been clam before the fall event. Someone who is intent on faking a fall would probably be nervous upfront, therefore having a higher breathing rate, and would not be as surprised after the event, which results in a lower difference in the breathing rate with respect to the pre-fall state.

The fall-event detection unit 108 of the fall-classification device 100 may also be additionally or alternatively configured to ascertain context-data indicative of a current context of the sensing volume, and to determine the fall-event further in dependence on predetermined associations between different contexts of the sensing volumes with an expected occurrence of the fall event. Thus, in addition to the subject-activity metrics that can dynamically be measured via RF sensing by the fall-classification device 100, the fall-classification arrangement 150, 250 can rely on other sources of context that can increase the likelihood that a possible event is indeed a fall. For example areas more prone to spills or wet surfaces (bathrooms/toilets, kitchens, machinery rooms, fountains, outdoor areas, especially if they are fitted with slippery tiles, isles in supermarket where liquids in glass bottles are stacked, etc.) are more likely locations for people slipping. Also, the type of action taking place in that area before/during the fall event can be used as context data to refine the fall-event determination. The types of action can further increase a likelihood of a fall event being real (e.g. in a bathroom in the middle of the day vs in a bathroom directly after a shower), or simply make any area potentially more hazardous (e.g. cleaning crew just finished in a certain aisle in a supermarket). Additionally, basic information like time of day (a person might pay less attention when going to the toilet at night than during the day since they are probably sleepy), environmental conditions (was there sufficient light to prevent stumbles), presence of sources of distractions (e.g. pets, kids that could leave toys lying around, etc.) can be used as context data for further refining the determination of the occurrence of the fall event or the type of fall event.

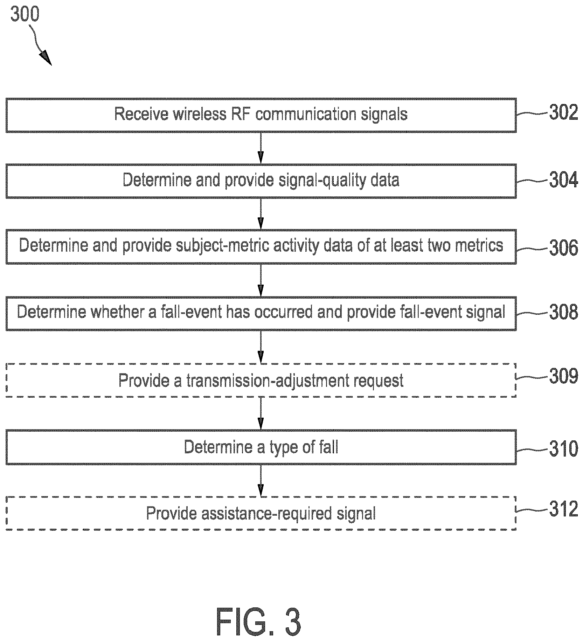

Fig. 3 shows a schematic flow diagram of a method 300 for operating a fall-classification device. The method comprises, in a step 302, receiving, at a receiver unit, wireless radiofrequency communication signals from one or more external wireless transmitters that, together with the receiver unit, define a respective sensing volume for sensing a plurality of subject-activity metrics, e.g., presence, motion, acceleration, gestures, gait, breathing rate, heart rate, etc., indicative of a respective subject activity of one or more subjects located within the sensing volume. The method also comprises, in a step 304, determining and providing a respective signal-quality value sequence including signal-quality values obtained at different points in time, the signal-quality values being indicative of a signal strength of the received wireless communication signal or being indicative of a channel state of a wireless communication link between a respective one of the external wireless transmitter and the receiver unit or being indicative of any other suitable signal-quality metric that is correlatable to a subject-activity. The method also comprises, in a step 306, determining and providing , using the provided signal-quality value sequence, respective subject-activity data indicative of at least two subject-activity metrics of the subject within the sensing volume. Further, the method comprises, in a step 308, determining, based on a time variation of at least one of the at least two subject-activity metrics, whether a fall-event of the subject within the sensing volume has occurred and providing a fall-event signal indicative thereof. The method also comprises, in a step 310, and upon reception of the fall-event-signal, determining, using a predetermined algorithm, a type of fall from a predetermined set of detectable types of fall using the subject-activity values of at least two subject-activity metrics determined during a first time span before the determination of the fall-event and during a second time span after the determination of the fall-event, and providing a fall-type signal indicative thereof.

The method 300 may optionally comprise, in a step 309, and upon determining the fall event, providing a transmission-adjustment request, indicative of:
- a request to the external wireless transmitters for providing the wireless communication signals with an increased transmission power; or
- a request to increase a number of external wireless transmitters that provide wireless communication signals for the determination of the signal-quality values; or
- a request to receive wireless communication signals from a predetermined set of external wireless transmitters; or any combination thereof.

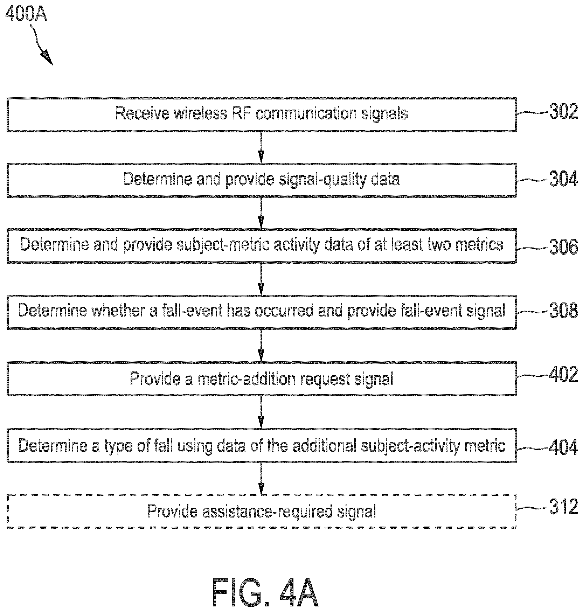

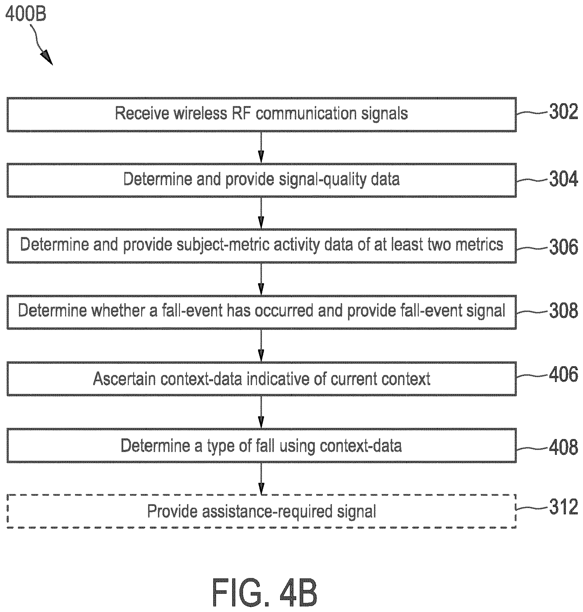

Fig. 4 shows a schematic flow diagram of another embodiment of a method 400 for operating a fall-classification device. The method 400 of Fig. 4 additionally comprises, in a step 402, providing, upon determination of a fall-event, a metric-addition request signal that is indicative of a request to determine subject-act values of an additional subject-parameter; and, in a step 404, determining the type of fall additionally using the subject-parameter values of the additional subject-parameter.

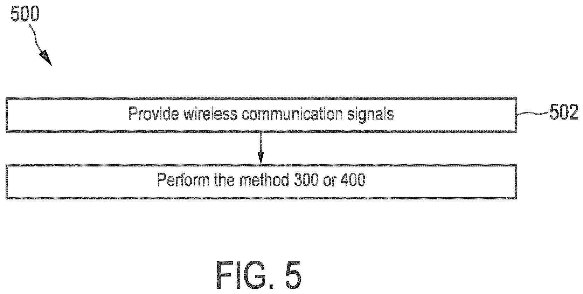

Fig. 5 shows a flow diagram of an embodiment of a method 500 for operating a fall-classification arrangement. The method comprises, in a step 502, providing wireless communication signals; and performing the method of Figs. 3 or 4.

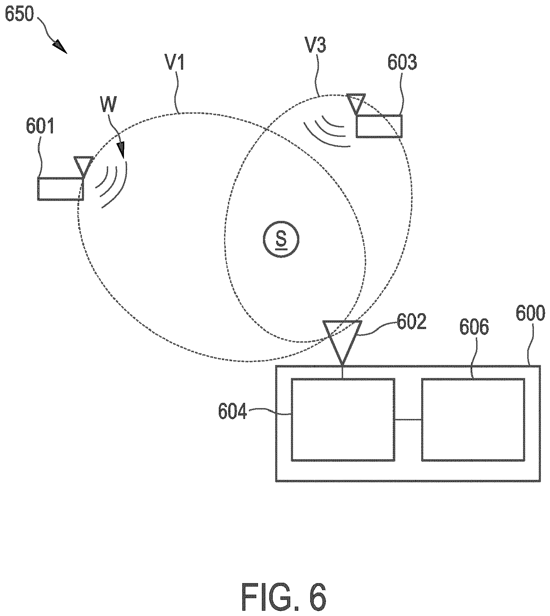

Fig. 6 shows a schematic diagram of an embodiment of a fall-determination device 600 forming part of a fall-determination arrangement 650. The fall-determination device comprises a receiver 602 unit configured to receive wireless radiofrequency communication signals W from one or more wireless transmitters 601, 603 of the arrangement 650 which, together with the receiver unit 602, define a respective sensing volume V1, V3 for sensing one or more subjects S located within the sensing volume. The fall-determination device 600 also comprises a signal quality determination unit 604 that is connected to the receiver unit 602 and that is configured to determine and provide a respective signal-quality value sequence including signal-quality values obtained at different points in time, the signal-quality values being indicative of a signal strength of the received wireless communication signal W or being indicative of a channel state of a wireless communication link between a respective one of the external wireless transmitter and the receiver unit or being indicative of any other suitable signal-quality metric that is correlatable to a fall movement of the subject S. The fall-determination device 600 also comprises a fall-event detection unit 606 that is connected to the signal quality determination unit 604 and configured to receive the signal-quality value sequence therefrom. The fall-event determination unit 606 is further configured to ascertain context-data indicative of a current context of the sensing volume V1, V3 and/or of the subject or subjects S located within the sensing volume and to determine, using the received signal-quality value sequence and the ascertained context-data whether a fall of a subject in the sensing volume has occurred.

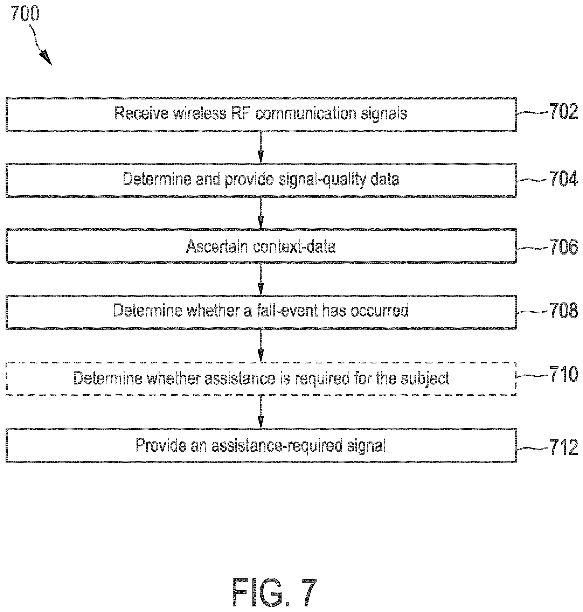

Fig. 7 shows a flow diagram of an embodiment of a method 700 for operating a fall-determination device. The method comprises, in a step 702, receiving wireless radiofrequency communication signals, in particular from one or more external wireless transmitters which, together with a receiver unit of the fall-determination device, define a respective sensing volume for sensing one or more subjects located within the sensing volume. The method comprises, in a step 704, determining and providing a respective signal-quality value sequence including signal-quality values obtained at different points in time, the signal-quality values being indicative of a signal strength of the received wireless communication signal or being indicative of a channel state of a wireless communication link between a respective one of the external wireless transmitter and the receiver unit of the fall-determination device or being indicative of any other suitable signal-quality metric that is correlatable to a subject-activity. The method comprises, in a step 706, ascertaining context-data indicative of a current context of the sensing volume and/or of the subjects located within the sensing volume. The method also comprises, in a step 708, determining, using the received signal-quality value sequence and the ascertained context-data whether a fall of a subject in the sensing volume has occurred.

The method 700 may optionally comprise the steps of determining 710 whether an assistance , e.g. medical assistance, is required for the person that has fallen and providing 712 an assistance-required signal indicative thereof, for instance to a medical or care institution.

In summary, the invention is directed to a fall-classification device with a receiver unit configured to receive wireless communication signals from wireless transmitters and a subject-activity data determination unit configured to determine and provide, using signal-quality values determined by a quality signal determination unit, respective subject-activity data indicative of at least two subject-activity metrics of a subject within a sensing volume. A fall-event detection unit is configured to determine, based on a time variation of the subject-activity data of at least one subject-activity metric, whether a fall-event has occurred. A fall-event type classification unit is configured, using a predetermined algorithm and subject-activity data of at least two subject-activity metrics determined during a first time span before and a second time span after the determination of the fall-event, to determine a fall type, from a predetermined list of fall types, with increased accuracy.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A fall-classification device (100), comprising:
- a receiver unit (102) configured to receive wireless radiofrequency communication signals (W) from one or more external wireless transceivers (101, 103) that, together with the receiver unit (102), define a respective sensing volume (V 1, V3) for sensing a plurality of subject-activity metrics indicative of a respective subject activity of one or more subjects (S) located within the sensing volume;
- a signal quality determination unit (104) connected to the receiver unit (102) and configured to determine and provide a respective signal-quality value sequence including signal-quality values obtained at different points in time, the signal-quality values being indicative of a signal strength of the received wireless communication signal or being indicative of a channel state of a wireless communication link between a respective one of the external wireless transceiver and the receiver unit or being indicative of any other suitable signal-quality metric that is correlatable to a subject-activity;
- a subject-activity data determination unit (106) configured to determine and provide, using the provided signal-quality value sequence, respective subject-activity data indicative of at least two subject-activity metrics of the subject (S) within the sensing volume (V1, V3);
- a fall-event detection unit (108) connected to the subject-activity data determination unit (106) and configured to receive the subject-activity data and, based on a time variation of the subject-activity data of at least one subject-activity metric, to determine whether a fall-event of the subject (S) within the sensing volume (V1, V3) has occurred and to provide a fall-event signal indicative thereof, wherein the fall-event detection unit (108) is configured, upon determination of the fall event, to provide to the external wireless transceivers (101, 103, 105, 107) a transmission-adjustment request, indicative of:
- a request to the external wireless transceivers for providing the wireless communication signals with an increased transmission power and/or to modify a transmission beam for transmitting the wireless communication signals, or
- a request to increase a number of external wireless transceivers that provide wireless communication signals for the determination of the signal-quality values, or
- a request to receive wireless communication signals from a predetermined set of external wireless transceivers,
- or any combination thereof; and
- a fall-event type classification unit (110) connected to the fall event-detection unit (108) and to the subject-activity data determination unit (106) and configured, upon reception of the fall-event-signal, to determine, using a predetermined algorithm, a type of fall from a predetermined set of detectable types of fall using the subject-activity data of at least two subject-activity metrics determined during a first time span before the determination of the fall-event and during a second time span after the determination of the fall-event, and to provide a fall-type signal indicative thereof.

2. The fall-classification device (100) of claim 1, wherein the fall-event detection unit comprises a storage unit (112) comprising fall-data that associates predetermined time variations of the subject-activity data of the respective subject-activity metrics with an expected occurrence of a fall event, and wherein the fall-event detection unit is configured to determine whether the fall-event has occurred using the stored fall data.

3. The fall-classification device (100) of claim 1 or 2, wherein the fall-event type classification unit comprises a storage unit (114) comprising fall-type data that associates predetermined combinations of time variations of two or more subject-activity data with a respective fall-type, and wherein the fall-event classification unit is configured to determine the type of fall using the stored fall-type data.

4. The fall-classification device (100) of any of the preceding claims, wherein
- the subject-activity data determination unit (106) is configured to be operable using at least a high sensitivity grade and a low sensitivity grade for determining the subject-activity data; wherein
- upon determination of a fall-event, the fall-event detection unit (108) is configured to provide to the subject-activity data determination unit (106) a sensitivity-increase request signal; and wherein
- the subject-activity data determination unit (106), upon reception of the sensitivity-increase request signal is configured to increase the sensitivity from the low sensitivity grade to the high sensitivity grade for determining the subject-activity data during the second time span.

5. The fall-classification device (100) of any of the preceding claims, wherein the fall-event detection unit (108) is configured, upon determination of a fall-event, to provide to the subject-activity data determination unit (106) a metric-addition request signal that is indicative of a request to determine subject-activity data of an additional subject-activity metric, and wherein the fall-event-type determination unit (110) is configured to determine the type of fall additionally using the subject-activity data of the additional subject-activity metric.

6. The fall-classification device (100) of any of the preceding claims, wherein the fall-event detection unit (108) is further configured to determine a fall-event context indicative of a duration of the fall-event of the subject within the sensing volume, or of a fall vertical distance, or of a fall end height, or of a maximum fall speed of the fall-event of the subject within the sensing volume and to provide a fall-context signal indicative thereof, and wherein the fall-event type classification unit (110) is further configured to determine the type of fall further using the provided fall-context signal.

7. The fall-classification device (100) of any of the preceding claims, wherein the fall-event detection unit (108) is further configured to ascertain context-data indicative of a current context of the sensing volume, and to determine the fall-event further in dependence on predetermined associations between different contexts of the sensing volumes with an expected occurrence of the fall event.

8. The fall-event classification device of any of the preceding claims, wherein the fall-event detection unit (108) is further configured to ascertain context-data indicative of a current context of the sensing volume and to determine, using the fall-type signal and the context-data whether an assistance is required for the fallen subject and to provide an assistance-required signal indicative thereof.

9. A fall-classification arrangement (150, 250) comprising:
- at least one fall-classification device (100) in accordance with any of the preceding claims; and
- one or more wireless transceivers (101, 103, 105, 107) configured to provide the wireless communication signals (W, W1, W3, W5, W7) to the at least one fall-classification device (100).

10. Method (300) for operating a fall-classification device, the method comprising:
- receiving (302), at a receiver unit, wireless radiofrequency communication signals from one or more external wireless transceivers that, together with the receiver unit, define a respective sensing volume for sensing a plurality of subject-activity metrics indicative of a respective subject activity of one or more subjects located within the sensing volume;
- determining and providing (304) a respective signal-quality value sequence including signal-quality values obtained at different points in time, the signal-quality values being indicative of a signal strength of the received wireless communication signal or being indicative of a channel state of a wireless communication link between a respective one of the external wireless transceiver and the receiver unit or being indicative of any other suitable signal-quality metric that is correlatable to a subject-activity;
- determining and providing (306), using the provided signal-quality value sequence, respective subject-activity data indicative of at least two subject-activity metrics of the subject within the sensing volume;
- based on a time variation of at least one of the at least two subject-activity metrics, determining (308) whether a fall-event of the subject within the sensing volume has occurred and providing a fall-event signal indicative thereof;
- upon determination of the occurance of the fall event, providing to the external wireless transceivers a transmission-adjustment request, indicative of:
- a request to the external wireless transceivers for providing the wireless communication signals with an increased transmission power and/or to modify a transmission beam for transmitting the wireless communication signals, or
- a request to increase a number of external wireless transceivers that provide wireless communication signals for the determination of the signal-quality values, or
- a request to receive wireless communication signals from a predetermined set of external wireless transceivers,
- or any combination thereof; and
- upon reception of the fall-event-signal, determining (310), using a predetermined algorithm, a type of fall from a predetermined set of detectable types of fall using the subject-activity data of at least two subject-activity metrics determined during a first time span before the determination of the fall-event and during a second time span after the determination of the fall-event, and providing a fall-type signal indicative thereof.

11. The method (400) of claim 10, further comprising:
- providing (402), upon determination of a fall-event, a metric-addition request signal that is indicative of a request to determine subject-act values of an additional subject-parameter; and
- determining (404) the type of fall additionally using the subject-parameter values of the additional subject-parameter.

12. A method (500) for operating a fall-classification arrangement, comprising:
- providing (502) wireless communication signals; and
- performing the method (300, 400) of any of the claims 10 to 11.

13. A computer program comprising instructions that, when executed by a computer, cause the computer to carry out the methods of any of the claims 10 to 12.

## Patentansprüche

1. Sturzklassifizierungsvorrichtung (100), umfassend:
- eine Empfängereinheit (102), die konfiguriert ist, um drahtlose Hochfrequenzkommunikationssignale (W) von einem oder mehreren externen drahtlosen Sendeempfängern (101, 103) zu empfangen, die, zusammen mit der Empfängereinheit (102), ein jeweiliges Erfassungsvolumen (V1, V3) zum Erfassen einer Vielzahl von Subjektaktivitätsmetriken definieren, die eine jeweilige Subjektaktivität von einem oder mehreren Subjekten (S) anzeigt, die sich innerhalb des Erfassungsvolumens befinden;
- eine Signalqualitätsbestimmungseinheit (104), die mit der Empfängereinheit (102) verbunden und konfiguriert ist, um eine jeweilige Signalqualitätswertfolge zu bestimmen und bereitzustellen, die Signalqualitätswerte einschließt, die zu unterschiedlichen Zeitpunkten erhalten werden, wobei die Signalqualitätswerte eine Signalstärke des empfangenen drahtlosen Kommunikationssignals anzeigen oder einen Kanalzustand einer drahtlosen Kommunikationsverknüpfung zwischen einem jeweiligen externen drahtlosen Sendeempfänger und der Empfängereinheit anzeigen oder eine beliebige andere geeignete Signalqualitätsmetrik anzeigen, die mit einer Subjektaktivität korrelierbar ist;
- eine Subjektaktivitätsdatenbestimmungseinheit (106), die konfiguriert ist, um, unter Verwendung der bereitgestellten Signalqualitätswertfolge, jeweilige Subjektaktivitätsdaten zu bestimmen und bereitzustellen, die mindestens zwei Subjektaktivitätsmetriken des Subjekts (S) innerhalb des Erfassungsvolumens (V1, V3) anzeigen;
- eine Sturzereigniserkennungseinheit (108), die mit der Subjektaktivitätsdatenbestimmungseinheit (106) verbunden und konfiguriert ist, um die Subjektaktivitätsdaten zu empfangen und, basierend auf einer Zeitvariation der Subjektaktivitätsdaten von mindestens einer Subjektaktivitätsmetrik, zu bestimmen, ob ein Sturzereignis des Subjekts (S) innerhalb des Erfassungsvolumens (V1, V3) aufgetreten ist und um ein Sturzereignissignal bereitzustellen, das dies anzeigt, wobei die Sturzereigniserkennungseinheit (108) konfiguriert ist, um, bei einer Bestimmung des Sturzereignisses, eine Übertragungsanpassungsanforderung an die externen drahtlosen Sendeempfänger (101, 103, 105, 107) bereitzustellen, die anzeigt:
- eine Aufforderung an die externen drahtlosen Sendeempfänger zum Bereitstellen der drahtlosen Kommunikationssignale mit einer erhöhten Übertragungsleistung und/oder einen Übertragungsstrahl zum Übertragen der drahtlosen Kommunikationssignale zu modifizieren, oder
- eine Aufforderung, die Anzahl der externen drahtlosen Sendeempfänger zu erhöhen, die drahtlose Kommunikationssignale für die Bestimmung der Signalqualitätswerte bereitstellen, oder
- eine Aufforderung, um drahtlose Kommunikationssignale von einem zuvor bestimmten Satz von externen drahtlosen Sendeempfängern zu empfangen,
- oder eine beliebige Kombination davon; und
- eine Sturzereignisartklassifizierungseinheit (110), die mit der Sturzereigniserkennungseinheit (108) und mit der Subjektaktivitätsdatenbestimmungseinheit (106) verbunden und konfiguriert ist, um, bei einem Empfang des Sturzereignissignals, unter Verwendung eines zuvor bestimmten Algorithmus, eine Art von Sturz von einem zuvor bestimmten Satz von erkennbaren Arten von Sturz zu bestimmen, unter Verwendung der Subjektaktivitätsdaten von mindestens zwei Subjektaktivitätsmetriken, die während einer ersten Zeitspanne vor der Bestimmung des Sturzereignisses und während einer zweiten Zeitspanne nach der Bestimmung des Sturzereignisses bestimmt wurden, und um ein Sturzartsignal bereitzustellen, das dies anzeigt.

2. Sturzklassifizierungsvorrichtung (100) nach Anspruch 1, wobei die Sturzereigniserkennungseinheit eine Speichereinheit (112) umfasst, umfassend Sturzdaten, die zuvor bestimmte Zeitvariationen der Subjektaktivitätsdaten der jeweiligen Subjektaktivitätsmetriken mit einem erwarteten Auftreten eines Sturzereignisses assoziieren, und wobei die Sturzereigniserkennungseinheit konfiguriert ist, um unter Verwendung der gespeicherten Sturzdaten zu bestimmen, ob das Sturzereignis aufgetreten ist.

3. Sturzklassifizierungsvorrichtung (100) nach Anspruch 1 oder 2, wobei die Sturzereignisklassifizierungseinheit eine Speichereinheit (114) umfasst, umfassend Sturzartdaten, die zuvor bestimmte Kombinationen von Zeitvariationen von zwei oder mehr Subjektaktivitätsdaten mit einer jeweiligen Sturzart assoziieren, und wobei die Sturzereignisklassifizierungseinheit konfiguriert ist, um die Art von Sturz unter Verwendung der gespeicherten Sturzartdaten zu bestimmen.

4. Sturzklassifizierungsvorrichtung (100) nach einem der vorstehenden Ansprüche, wobei
- die Subjektaktivitätsdatenbestimmungseinheit (106) konfiguriert ist, um unter Verwendung mindestens eines hohen Empfindlichkeitsgrades und eines niedrigen Empfindlichkeitsgrades zum Bestimmen der Subjektaktivitätsdaten betriebsfähig zu sein; wobei
- bei der Bestimmung eines Sturzereignisses, die Sturzereigniserkennungseinheit (108) konfiguriert ist, um ein Empfindlichkeitserhöhungsanforderungssignal bereitzustellen an die Subjektaktivitätsdatenbestimmungseinheit (106); und wobei
- die Subjektaktivitätsdatenbestimmungseinheit (106), bei dem Empfang des Empfindlichkeitserhöhungsanforderungssignals konfiguriert ist, um die Empfindlichkeit von dem niedrigen Empfindlichkeitsgrad auf den hohen Empfindlichkeitsgrad zum Bestimmen der Subjektaktivitätsdaten während der zweiten Zeitspanne zu erhöhen.

5. Sturzklassifizierungsvorrichtung (100) nach einem der vorstehenden Ansprüche, wobei die Sturzereigniserkennungseinheit (108) konfiguriert ist, um, bei der Bestimmung eines Sturzereignisses, ein Metrikzusatzanforderungssignal an die Subjektaktivitätsdatenbestimmungseinheit (106) bereitzustellen, das eine Anforderung anzeigt, um Subjektaktivitätsdaten einer zusätzlichen Subjektaktivitätsmetrik zu bestimmen, und wobei die Sturzereignisartbestimmungseinheit (110) konfiguriert ist, um die Art von Sturz zusätzlich unter Verwendung der Subjektaktivitätsdaten der zusätzlichen Subjektaktivitätsmetrik zu bestimmen.

6. Sturzklassifizierungsvorrichtung (100) nach einem der vorstehenden Ansprüche, wobei die Sturzereigniserkennungseinheit (108) ferner konfiguriert ist, um einen Sturzereigniskontext zu bestimmen, der eine Dauer des Sturzereignisses des Subjekts innerhalb des Erfassungsvolumens oder eine vertikale Sturzdistanz oder eine Sturzendhöhe oder eine maximale Sturzgeschwindigkeit des Sturzereignisses des Subjekts innerhalb des Erfassungsvolumens anzeigt, und um ein Sturzkontextsignal bereitzustellen, das dies anzeigt, und wobei die Sturzereignisartklassifizierungseinheit (110) ferner konfiguriert ist, um die Art von Sturz unter Verwendung des bereitgestellten Sturzkontextsignals ferner zu bestimmen.

7. Sturzklassifizierungsvorrichtung (100) nach einem der vorstehenden Ansprüche, wobei die Sturzereigniserkennungseinheit (108) ferner konfiguriert ist, um Kontextdaten zu ermitteln, die einen aktuellen Kontext des Erfassungsvolumens anzeigen, und um das Sturzereignis ferner in Abhängigkeit von zuvor bestimmten Assoziationen zwischen unterschiedlichen Kontexten der Erfassungsvolumina mit einem erwarteten Auftreten des Sturzereignisses zu bestimmen.

8. Sturzereignisklassifizierungsvorrichtung nach einem der vorstehenden Ansprüche, wobei die Sturzereigniserkennungseinheit (108) ferner konfiguriert ist, um Kontextdaten zu ermitteln, die einen aktuellen Kontext des Erfassungsvolumens anzeigen, und um unter Verwendung des Sturzartsignals und der Kontextdaten zu bestimmen, ob eine Hilfe für das gestürzte Subjekt erforderlich ist, und um ein Hilfe-erforderlich-Signal bereitzustellen, das dies anzeigt.

9. Sturzklassifizierungsanordnung (150, 250), umfassend:
- mindestens eine Sturzklassifizierungsvorrichtung (100) nach einem der vorstehenden Ansprüche; und
- einen oder mehrere drahtlose Sendeempfänger (101, 103, 105, 107), die konfiguriert sind, um die drahtlosen Kommunikationssignale (W, W1, W3, W5, W7) an die mindestens eine Sturzklassifizierungsvorrichtung (100) bereitzustellen.

10. Verfahren (300) zum Betreiben einer Sturzklassifizierungsvorrichtung, das Verfahren umfassend:
- Empfangen (302), an einer Empfängereinheit, von drahtlosen Hochfrequenzkommunikationssignalen von einem oder mehreren externen drahtlosen Sendeempfängern, die, zusammen mit der Empfängereinheit, ein jeweiliges Erfassungsvolumen zum Erfassen einer Vielzahl von Subjektaktivitätsmetriken definieren, die eine jeweilige Subjektaktivität von einem oder mehreren Subjekten anzeigt, die sich innerhalb des Erfassungsvolumens befinden;
- Bestimmen und Bereitstellen (304) einer jeweiligen Signalqualitätswertfolge, die Signalqualitätswerte einschließt, die zu unterschiedlichen Zeitpunkten erhalten wurden, wobei die Signalqualitätswerte eine Signalstärke des empfangenen drahtlosen Kommunikationssignals anzeigen oder einen Kanalzustand einer drahtlosen Kommunikationsverknüpfung zwischen einem jeweiligen externen drahtlosen Sendeempfänger und der Empfängereinheit anzeigen oder eine beliebige andere geeignete Signalqualitätsmetrik anzeigen, die mit einer Subjektaktivität korrelierbar ist;
- Bestimmen und Bereitstellen (306), unter Verwendung der bereitgestellten Signalqualitätswertfolge, von jeweiligen Subjektaktivitätsdaten, die mindestens zwei Subjektaktivitätsmetriken des Subjekts innerhalb des Erfassungsvolumens anzeigen;
- basierend auf einer Zeitvariation von mindestens einer der mindestens zwei Subjektaktivitätsmetriken, Bestimmen (308), ob ein Sturzereignis des Subjekts innerhalb des Erfassungsvolumens aufgetreten ist, und Bereitstellen eines Sturzereignissignals, das dies anzeigt;
- bei der Bestimmung des Auftretens des Sturzereignisses, Bereitstellen einer Übertragungsanpassungsanforderung an die externen drahtlosen Sendeempfänger, die anzeigt:
- eine Aufforderung an die externen drahtlosen Sendeempfänger zum Bereitstellen der drahtlosen Kommunikationssignale mit einer erhöhten Übertragungsleistung und/oder einen Übertragungsstrahl zum Übertragen der drahtlosen Kommunikationssignale zu modifizieren, oder
- eine Aufforderung, die Anzahl der externen drahtlosen Sendeempfänger zu erhöhen, die drahtlose Kommunikationssignale für die Bestimmung der Signalqualitätswerte bereitstellen, oder
- eine Aufforderung, um drahtlose Kommunikationssignale von einem zuvor bestimmten Satz von externen drahtlosen Sendeempfängern zu empfangen,
- oder eine beliebige Kombination davon; und
- bei dem Empfang des Sturzereignissignals, Bestimmen (310), unter Verwendung eines zuvor bestimmten Algorithmus, einer Art von Sturz von einem zuvor bestimmten Satz von erkennbaren Arten von Sturz unter Verwendung der Subjektaktivitätsdaten von mindestens zwei Subjektaktivitätsmetriken, die während einer ersten Zeitspanne vor der Bestimmung des Sturzereignisses und während einer zweiten Zeitspanne nach der Bestimmung des Sturzereignisses bestimmt wurden, und Bereitstellen eines Sturzartsignals, das dies anzeigt.

11. Verfahren (400) nach Anspruch 10, ferner umfassend:
- Bereitstellen (402), bei der Bestimmung eines Sturzereignisses, eines Metrikzusatzanforderungssignals, das eine Anforderung anzeigt, um Subjektaktwerte eines zusätzlichen Subjekparameters zu bestimmen; und
- Bestimmen (404) der Art von Sturz zusätzlich unter Verwendung der Subjektparameterwerte des zusätzlichen Subjektparameters.

12. Verfahren (500) zum Betreiben einer Sturzklassifizierungsanordnung, umfassend:
- Bereitstellen (502) von drahtlosen Kommunikationssignalen; und
- Durchführen des Verfahrens (300, 400) nach einem der Ansprüche 10 bis 11.

13. Computerprogramm, umfassend Anweisungen, die, wenn sie durch einen Computer ausgeführt wird, den Computer veranlassen, die Verfahren nach einem der Ansprüche 10 bis 12 vorzunehmen.

## Revendications

1. Dispositif de classification de chute (100), comprenant :
- une unité réceptrice (102) configurée pour recevoir des signaux de communication radiofréquence sans fil (W) depuis un ou plusieurs émetteurs-récepteurs sans fil externes (101, 103) qui, conjointement avec l'unité réceptrice (102), définissent un volume de détection (V1, V3) respectif pour la détection d'une pluralité de métriques d'activité de sujet indiquant une activité de sujet respective d'un ou plusieurs sujets (S) situés au sein du volume de détection ;
- une unité de détermination de qualité de signal (104) connectée à l'unité réceptrice (102) et configurée pour déterminer et fournir une séquence respective de valeurs de qualité de signal comportant des valeurs de qualité de signal obtenues à différents points dans le temps, les valeurs de qualité de signal indiquant une intensité de signal du signal de communication sans fil reçu ou indiquant un état de canal d'une liaison de communication sans fil entre l'un respectif de l'émetteur-récepteur sans fil externe et de l'unité réceptrice ou indiquant toute autre métrique de qualité de signal appropriée qui est corrélable à une activité de sujet ;
- une unité de détermination de données d'activité de sujet (106) configurée pour déterminer et fournir, à l'aide de la séquence de valeurs de qualité de signal fournie, des données d'activité de sujet respectives indiquant au moins deux métriques d'activité de sujet du sujet (S) au sein du volume de détection (V1, V3) ;
- une unité de détection d'événement de chute (108) connectée à l'unité de détermination de données d'activité de sujet (106) et configurée pour recevoir les données d'activité de sujet et, en fonction d'une variation temporelle des données d'activité de sujet d'au moins une métrique d'activité de sujet, pour déterminer si un événement de chute du sujet (S) au sein du volume de détection (V1, V3) est survenu et pour fournir un signal d'événement de chute indiquant celui-ci, dans lequel l'unité de détection d'événement de chute (108) est configurée, lors de la détermination de l'événement de chute, pour fournir aux émetteurs-récepteurs sans fil externes (101, 103, 105, 107) une demande d'ajustement de transmission, indiquant :
- une demande aux émetteurs-récepteurs sans fil externes pour la fourniture des signaux de communication sans fil avec une puissance de transmission augmentée et/ou pour la modification d'un faisceau de transmission pour la transmission des signaux de communication sans fil, ou
- une demande pour augmenter un nombre d'émetteurs-récepteurs sans fil externes qui fournissent des signaux de communication sans fil pour la détermination des valeurs de qualité de signal, ou
- une demande pour recevoir des signaux de communication sans fil à partir d'un ensemble prédéterminé d'émetteurs-récepteurs sans fil externes,
- ou l'une quelconque combinaison de ceux-ci ; et
- une unité de classification de type d'événement de chute (110) connectée à l'unité de détection d'événement de chute (108) et à l'unité de détermination de données d'activité de sujet (106) et configurée, lors de la réception du signal d'événement de chute, pour déterminer, à l'aide d'un algorithme prédéterminé, un type de chute à partir d'un ensemble prédéterminé de types détectables de chute à l'aide des données d'activité de sujet d'au moins deux mesures d'activité de sujet déterminées pendant une première période de temps avant la détermination de l'événement de chute et pendant une seconde période de temps après la détermination de l'événement de chute, et pour fournir un signal de type de chute indiquant celui-ci.

2. Dispositif de classification de chute (100) selon la revendication 1, dans lequel l'unité de détection d'événement de chute comprend une unité de stockage (112) comprenant des données de chute qui associent des variations de temps prédéterminées des données d'activité de sujet des métriques d'activité de sujet respectives à une survenue attendue d'un événement de chute, et dans lequel l'unité de détection d'événement de chute est configurée pour déterminer si l'événement de chute est survenu à l'aide des données de chute stockées.

3. Dispositif de classification de chute (100) selon la revendication 1 ou 2, dans lequel l'unité de classification de type d'événement de chute comprend une unité de stockage (114) comprenant des données de type de chute qui associent des combinaisons prédéterminées de variations de temps de deux ou plusieurs données d'activité de sujet avec un type de chute respectif, et dans lequel l'unité de classification d'événement de chute est configurée pour déterminer le type de chute à l'aide des données de type d'événement de chute stockées.

4. Dispositif de classification de chute (100) selon l'une quelconque des revendications précédentes, dans lequel
- l'unité de détermination de données d'activité de sujet (106) est configurée pour pouvoir fonctionner à l'aide d'au moins un niveau de sensibilité élevée et d'un niveau de sensibilité faible pour déterminer les données d'activité de sujet ; dans lequel
- lors de la détermination d'un événement de chute, l'unité de détection d'événement de chute (108) est configurée pour fournir à l'unité de détermination de données d'activité de sujet (106) un signal de demande d'augmentation de sensibilité ; et dans lequel
- l'unité de détermination de données d'activité de sujet (106), lors de la réception du signal de demande d'augmentation de sensibilité est configurée pour augmenter la sensibilité du niveau de sensibilité faible au niveau de sensibilité élevée pour la détermination des données d'activité de sujet pendant la seconde période de temps.

5. Dispositif de classification de chute (100) selon l'une quelconque des revendications précédentes, dans lequel l'unité de détection d'événement de chute (108) est configurée, lors de la détermination d'un événement de chute, pour fournir à l'unité de détermination de données d'activité de sujet (106) un signal de demande d'ajout de métrique indiquant une demande pour déterminer des données d'activité de sujet d'une métrique d'activité de sujet supplémentaire, et dans lequel l'unité de détermination de type d'événement de chute (110) est configurée pour déterminer le type de chute à l'aide en complément des données d'activité de sujet de la métrique d'activité de sujet supplémentaire.

6. Dispositif de classification de chute (100) selon l'une quelconque des revendications précédentes, dans lequel l'unité de détection d'événement de chute (108) est en outre configurée pour déterminer un contexte d'événement de chute indiquant une durée de l'événement de chute du sujet au sein du volume de détection, ou une distance verticale de chute, ou une hauteur de fin de chute, ou une vitesse de chute maximale de l'événement de chute du sujet au sein du volume de détection et pour fournir un signal de contexte de chute indiquant celui-ci, et dans lequel l'unité de classification de type d'événement (110) est en outre configurée pour déterminer le type de chute à l'aide en outre du signal de contexte de chute fourni.

7. Dispositif de classification de chute (100) selon l'une quelconque des revendications précédentes, dans lequel l'unité de détection d'événement de chute (108) est en outre configurée pour vérifier des données de contexte indiquant un contexte actuel du volume de détection, et pour déterminer l'événement de chute dépendant en outre d'associations prédéterminées entre différents contextes des volumes de détection avec une survenue attendue de l'événement de chute.

8. Dispositif de classification d'événement de chute selon l'une quelconque des revendications précédentes, dans lequel l'unité de détection d'événement de chute (108) est en outre configurée pour vérifier des données de contexte indiquant un contexte actuel du volume de détection et pour déterminer, à l'aide du signal de type de chute et des données de contexte, si une assistance est requise pour le sujet tombé et pour fournir un signal requis d'assistance indiquant celui-ci.

9. Agencement de classification de chute (150, 250) comprenant :
- au moins un dispositif de classification de chute (100) conformément à l'une quelconque des revendications précédentes ; et
- un ou plusieurs émetteurs-récepteurs sans fil (101, 103, 105, 107) configurés pour fournir les signaux de communication sans fil (W, W1, W3, W5, W7) à l'au moins un dispositif de classification de chute (100).

10. Procédé (300) permettant de faire fonctionner un dispositif de classification de chute, le procédé comprenant :
- la réception (302), au niveau d'une unité réceptrice, de signaux de communication radiofréquence sans fil provenant d'un ou plusieurs émetteurs-récepteurs sans fil externes qui, conjointement avec l'unité réceptrice, définissent un volume de détection respectif pour la détection d'une pluralité de métriques d'activité de sujet indiquant une activité de sujet respective d'un ou plusieurs sujets situés au sein du volume de détection ;
- la détermination et la fourniture (304) d'une séquence de valeurs de qualité de signal respective comportant des valeurs de qualité de signal obtenues à différents points dans le temps, les valeurs de qualité de signal indiquant une intensité de signal du signal de communication sans fil reçu ou indiquant un état de canal d'une liaison de communication sans fil entre l'un respectif de l'émetteur-récepteur sans fil externe et de l'unité réceptrice ou indiquant toute autre métrique de qualité de signal appropriée qui est corrélable à une activité de sujet ;
- la détermination et la fourniture (306), à l'aide de la séquence de valeurs de qualité de signal fournie, de données d'activité de signal respectives indiquant au moins deux métriques d'activité de sujet du sujet au sein du volume de détection ;
- en fonction d'une variation de temps d'au moins l'une des au moins deux métriques d'activité de sujet, la détermination (308) de si un événement de chute du sujet au sein du volume de détection est survenu et la fourniture d'un signal d'événement de chute indiquant celui-ci ;
- lors de la détermination de la survenue de l'événement de chute, la fourniture aux émetteurs-récepteurs sans fil externes d'une demande d'ajustement de transmission, indiquant :
- une demande aux émetteurs-récepteurs sans fil externes pour la fourniture des signaux de communication sans fil avec une puissance de transmission augmentée et/ou pour la modification d'un faisceau de transmission pour la transmission des signaux de communication sans fil, ou
- une demande pour augmenter un nombre d'émetteurs-récepteurs sans fil externes qui fournissent des signaux de communication sans fil pour la détermination des valeurs de qualité de signal, ou
- une demande pour recevoir des signaux de communication sans fil à partir d'un ensemble prédéterminé d'émetteurs-récepteurs sans fil externes,
- ou l'une quelconque combinaison de ceux-ci ; et
- lors de la réception du signal d'événement de chute, la détermination (310), à l'aide d'un algorithme prédéterminé, d'un type de chute d'un ensemble prédéterminé de types de chute détectables à l'aide des données d'activité de sujet d'au moins deux métriques d'activité de sujet déterminées pendant une première période de temps avant la détermination de l'événement de chute et pendant un second intervalle de temps après la détermination de l'événement de chute, et la fourniture d'un signal de type de chute indiquant celui-ci.

11. Procédé (400) selon la revendication 10, comprenant en outre :
- la fourniture (402), lors de la détermination d'un événement de chute, d'un signal de demande d'ajout de métrique indiquant une demande pour déterminer des valeurs d'action de sujet d'un paramètre de sujet complémentaire ; et
- la détermination (404) du type de chute à l'aide en complément des valeurs de paramètre de sujet du paramètre de sujet supplémentaire.

12. Procédé (500) permettant de faire fonctionner un dispositif de classification de chute comprenant :
- la fourniture (502) de signaux de communication sans fil ; et
- la mise en oeuvre du procédé (300, 400) selon l'une quelconque des revendications 10 à 11.

13. Programme d'ordinateur comprenant des instructions qui, lorsqu'il est exécuté par un ordinateur, amènent l'ordinateur à effectuer les procédés selon l'une quelconque des revendications 10 à 12.
